# EUROPÄISCHE PATENTSCHRIFT

(11) **EP 3 692 976 B1**
(45) Veröffentlichungstag und Bekanntmachung des Hinweises auf die Patenterteilung: **31.03.2021**
(21) Anmeldenummer: 19156084.6
(22) Anmeldetag: 07.02.2019
(51) Int. Cl.: A61K 6/17, A61K 6/79, A61K 6/816, A61K 6/818, A61K 6/887, C07C 409/12

(54) **DENTALMATERIALIEN AUF BASIS VON REDOXSYSTEMEN MIT GERUCHSARMEN CUMOLHYDROPEROXIDDERIVATEN**
DENTAL MATERIALS BASED ON REDOX SYSTEMS WITH LOW ODOR CUMENE HYDROPEROXIDE DERIVATIVES
MATIÈRES DENTAIRES À BASE DE SYSTÈMES REDOX À DÉRIVÉS DE HYDROPEROXYDE DE CUMÈNE PEU ODORANT

(43) Veröffentlichungstag der Anmeldung: 12.08.2020
(73) Patentinhaber: Ivoclar Vivadent AG, 9494 Schaan (LI)
(72) Erfinder: MOSZNER, Norbert, 9495 Triesen (LI); KÖHLER, Thomas, 78479 Reichenau (DE); SCHÄDLICH, Johannes, 9430 St. Magareten (CH); ANGERMANN, Jörg, 7320 Sargans (CH)
(74) Vertreter: Uexküll & Stolberg

(56) Entgegenhaltungen:
- EP-A1- 1 754 465
- EP-B1- 1 693 046
- WO-A1-2007/016508
- DE-C2- 2 635 595
- US-A1- 2003 134 933

## Beschreibung

Die vorliegende Erfindung betrifft radikalisch polymerisierbare Zusammensetzungen mit einem Cumolhydroperoxid-Redoxinitiatorsystem, das geruchsarme Cumolhydroperoxid-Derivate enthält. Die Zusammensetzungen eignen sich besonders als Dentalmaterialien, beispielsweise als Prothesenmaterialien, Zemente, Adhäsive und Komposite für direkte Füllungen.

Haupteinsatzgebiete von Polymeren im Dentalbereich sind die abnehmbare (z.B. Zähne und Prothesenbasismaterialien) und festsitzende Prothetik (z.B. Verblendmaterialen, Kronen oder Zemente), Füllungsmaterialien (z.B. direkte oder indirekte Füllungskomposite, Befestigungszemente oder Adhäsive) oder Hilfsmaterialien (z.B. Abformmaterialien). Die Polymeren werden gewöhnlich durch radikalische Polymerisation geeigneter Zusammensetzungen erhalten, die eine polymerisierbare organische Matrix enthalten, in der Regel eine Mischung von Monomeren, Initiator-Komponenten und Stabilisatoren.

Als Monomere werden meistens Methylmethacrylat (MMA) (Prothesenmaterialien), Mischungen von funktionalisierten Monomeren, wie z.B. 2-Hydroxyethylmethacrylat (HEMA), oder säuregruppenhaltigen Haftmonomeren, wie z.B. 10-Methacryloyloxydecyldihydrogenphosphat (MDP), mit Dimethacrylaten (Adhäsive) oder Mischungen eingesetzt, die ausschließlich Dimethacrylate enthalten (Kompositzemente und Füllungskomposite). Häufig verwendete Dimethacrylate sind 2,2-Bis[4-(2-hydroxy-3-methacryloyloxypropyl)phenyl]propan (Bis-GMA) und 1,6-Bis-[2-methacryloyloxy-ethoxycarbonylamino]-2,2,4-trimethylhexan (UDMA), die eine hohe Viskosität haben und zu Polymerisaten mit sehr guten mechanischen Eigenschaften führen. Als reaktive Verdünner finden vor allem Triethylenglycoldimethacrylat (TEGDMA), 1,10-Decandioldimethacrylat (D3MA) oder Bis(3-methacryloyloxymethyl)tricyclo-[5.2.1.02.6]decan (DCP) Anwendung.

Die Aushärtung von methacrylatbasierenden Dentalmaterialien erfolgt durch radikalische Polymerisation, wobei je nach Anwendungsgebiet radikalische Photoinitiatoren (Lichthärtung, direkte Füllungskomposite und Adhäsive), thermische Initiatoren (indirekte Komposite oder Prothesenmaterialien) oder Redox-Initiatorsysteme (Kompositzemente) eingesetzt werden und wobei auch die Kombination von Photoinitiatoren mit Redoxinitiatoren, z.B. bei Füllungen tiefer Kavitäten, bekannt ist.

Redoxsysteme kommen vor allem dann zum Einsatz, wenn z.B. bei Prothesenmaterialien wegen einer geringen Monomerreaktivität oder bei Befestigungszementen aufgrund unzureichender Durchstrahlung eine unvollständige Aushärtung zu befürchten ist.

Um eine ausreichende Lagerstabilität der Materialien zu gewährleisten, werden Werkstoffe auf der Basis von Redoxinitiatoren meist als sogenannte ZweiKomponenten-Systeme (2K-Systeme) eingesetzt, wobei das Oxydationsmittel (Peroxid- oder Hydroperoxid) und das Reduktionsmittel (Amine, Sulfinsäuren, Barbiturate, Thioharnstoff etc.) in zwei getrennte Komponenten eingearbeitet werden. Diese Komponenten werden kurz vor der Anwendung miteinander gemischt. Dabei müssen die beiden Komponenten so abgestimmt sein, dass ihre homogene Vermischung und Anwendung einfach möglich ist und dass eine für dentale Zwecke ausreichende Verarbeitungszeit erreicht wird. Unter der Verarbeitungszeit wird die Zeitspanne zwischen dem Vermischen der beiden Komponenten und der beginnenden Aushärtung des gemischten Materials verstanden. Diese sollte in einem Bereich von etwa 90 und 150 s liegen. Andererseits darf die Aushärtungszeit, d.h. der Zeitraum bis zur vollständigen Härtung der Materialen, nicht zu lang sein. Eine Aushärtungszeit von ca. 3 bis 5 min ist optimal.

Für dentale Kompositzemente wurden für lange Zeit vor allem Redoxinitiatorsysteme eingesetzt, die auf einer Mischung von Dibenzoylperoxid (DBPO) mit tertiären aromatischen Aminen, wie z.B. N,N-Diethanol-p-toluidin (DEPT), N,N-Dimethyl-sym.-xylidin (DMSX) oder N,N-Diethyl-3,5-di-tert.-butylanilin (DABA) beruhen. Mit DBPO/Amin-basierenden Redoxinitiatorsystemen lassen sich die Verarbeitungs- und Aushärtezeit in Kombination mit phenolischen Inhibitoren relativ gut einstellen. Ein Nachteil solcher DBPO/Amin-Systeme sind Verfärbungen, die durch eine langsame Oxidation der Amine verursacht werden. Außerdem wird die Radikalbildung bei DBPO/Amin-basierenden Redoxinitiatorsystemen durch Säuren und damit auch durch saure Monomere beeinträchtigt, die regelmäßig zur Herstellung von Schmelz-Dentin-Adhäsiven eingesetzt werden. Die Aminkomponente wird durch eine Säure-Base-Reaktion protoniert und dadurch deaktiviert.

Die voranstehenden Nachteile können mit Hydroperoxid-Redoxinitiatorsystemen zum Teil überwunden werden, weil keine tertiären Amine als Reduktionsmittel erforderlich sind. Außerdem sind Hydroperoxide thermisch stabiler als Peroxide. Cumolhydroperoxid weist beispielsweise eine 10-Stunden-Halbwertstemperatur T_{1/2} von 158 °C auf, die 10-Stunden-Halbwertstemperatur T_{1/2} von DBPO beträgt nur 73 °C.

Die DE 26 35 595 C2 offenbart polymerisierbare Zahnfüllmassen, die als Initiatorsystem ein substituiertes Thioharnstoff-Reduktionsmittel in Kombination mit einem Hydroperoxid-Oxydationsmittel enthalten. Die Materialien sollen eine verbesserte Farbbeständigkeit, eine hervorragende Härtegeschwindigkeit und eine verbesserte Lagerfähigkeit aufweisen.

Die EP 1 693 046 B1 offenbart dentale Zemente und Stumpfaufbaumaterialien, die ein (2-Pyridyl)-2-thioharnstoffderivat in Kombination mit einem Hydroperoxid enthalten, in dem die Hydroperoxidgruppe an ein tertiäres Kohlenstoffatom gebunden ist.

Die WO 2007/016508 A1 offenbart eine polymerisierbare dentale Zusammensetzung, die als Initiatorsystem ein Thioharnstoffderivat in Kombination mit einem Hydroperoxid enthält. Die Zusammensetzung enthält keine Monomeren mit Säuregruppen.

Gemäß EP 1 754 465 B1 lässt sich die Reaktivität des Cumolhydroperoxid/Acetylthioharnstoff-Systems durch die Zugabe von löslichen Kupferverbindungen steigern.

Die US 7,275,932 B2 schlägt die Verwendung von Hydroperoxiden und Thioharnstoffderivaten in Kombination mit einer sauren Verbindung als Beschleuniger vor. Bevorzugte saure Verbindungen sind Acrylate und Methacrylate mit Säuregruppen wie z.B. Methacrylsäure.

Die EP 2 233 544 A1 und die EP 2 258 336 A1 offenbaren Dentalwerkstoffe, die ein Hydroperoxid und ein Thioharnstoffderivat in Kombination mit einer Vanadiumverbindung als Beschleuniger enthalten.

Initiatorsysteme auf der Basis von Hydroperoxiden und insbesondere auf der Basis von Cumolhydroperoxid haben eine erhebliche Bedeutung bei der Vermeidung der mit Peroxid/Aminsystemen verbundenen Nachteile erlangt. Nachteilig an Cumolhydroperoxid ist sein typisch aromatischer Geruch, der an Xylole und Toluol erinnert und der vor allem bei Materialien für die intraorale Anwendung als unangenehm empfunden wird.

Der Erfindung liegt die Aufgabe zugrunde, Dentalmaterialien zur Verfügung zu stellen, die nicht die Nachteile des Standes der Technik aufweisen. Die Materialien sollen geruchsarm sein, über eine hohe Lagerstabilität verfügen und keine Verfärbungen zeigen, gleichzeitig aber schnell härten und dennoch eine für dentale Zwecke geeignete Verarbeitungszeit aufweisen. Die Materialen sollen weiterhin über gute mechanische Eigenschaften verfügen.

Diese Aufgabe wird durch radikalisch polymerisierbare Dentalwerkstoffe gelöst, die als Initiatorsystem für die radikalische Polymerisation eine Kombination aus einem Thioharnstoffderivat und einem Hydroperoxid gemäß der folgenden Formel (I) enthalten, 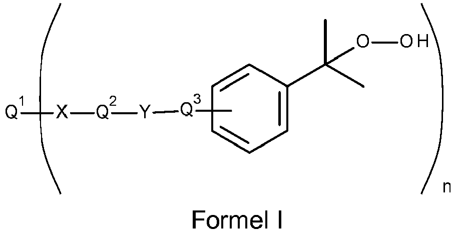 in der die Variablen die folgenden Bedeutungen haben:
- Q¹: ein n-wertiger, aromatischer, aliphatischer, linearer oder verzweigter C₁-C₁₄-Kohlenwasserstoffrest, der durch ein oder mehrere S- und/oder O-Atome unterbrochen sein kann und der unsubstituiert oder durch einen oder mehrere Substituenten, die vorzugsweise aus -OH, -OR¹, -Cl und -Br ausgewählt sind, substituiert sein kann, wobei R¹ ein aliphatischer, linearer oder verzweigter C₁-C₁₀-Kohlenwassrstoffrest ist,
- X, Y: unabhängig voneinander jeweils entfällt, -O-, -COO-; -CONR²-, oder -O-CO-NR³-, wobei R² und R³ unabhängig voneinander für H oder einen C₁-C₅-Alkylrest, vorzugsweise für H, Methyl und/oder Ethyl, besonders bevorzugt H stehen, und wobei X und Y nicht gleichzeitig entfallen,
- Q²: entfällt, ein aliphatischer, linearer oder verzweigter C₁-C₁₄-Alkylen-Rest, der durch S- und/oder O-Atome unterbrochen sein kann und der unsubstituiert oder durch -OH, -OR⁴, -Cl und/oder -Br substituiert sein kann, wobei R⁴ ein aliphatischer, linearer oder verzweigter C₁-C₁₀-Kohlenwasserstoffrest ist,
- Q³: eine C₁-C₃-Alkylengruppe oder entfällt, vorzugsweise -CH₂- oder entfällt, wobei X oder Y entfällt, wenn Q² entfällt,
- n: 1, 2, 3 oder 4, und wobei
die Substitution am Aromaten in 2-, 3- oder 4-Stellung erfolgt, relativ zur Cumolhydroperoxidgruppe.

Die Formel I erstreckt sich nur auf solche Verbindungen, die mit der chemischen Valenzlehre vereinbar sind. Der Hinweis, dass ein Rest z.B. durch ein oder mehrere O-Atome unterbrochen ist, ist so zu verstehen, dass diese Atome jeweils in die Kohlenstoffkette des Restes eingeschoben werden. Diese Atome sind damit beidseitig durch C-Atome begrenzt und können nicht endständig sein. C₁-Reste können nicht unterbrochen sein. Die Gruppen -COO-; -CONR²-, und -O-CO-NR³- können in beliebiger Orientierung angeordnet sein. Beispielsweise steht -COO- sowohl für -CO-O-als auch für -O-CO-. Unter aromatischen Kohlenwasserstoffresten werden der üblichen Nomenklatur entsprechend auch solche Reste verstanden, die aromatische und nicht aromatische Gruppen enthalten. Ein bevorzugter aromatischer Rest ist beispielsweise der p-Isopropylphenylrest.

Vorzugsweise haben die Variablen die folgenden Bedeutungen:
- Q¹: ein 1- oder 2-wertiger, aliphatischer, linearer oder verzweigter C₁-C₁₀-Kohlenwasserstoffrest, der durch ein oder mehrere O-Atome, vorzugsweise ein O-Atom, unterbrochen sein kann und der durch einen oder mehrere, vorzugsweise einen, Substituenten, die aus -OH und -OR¹ ausgewählt sind, substituiert sein kann oder vorzugsweise unsubstituiert ist, wobei R¹ ein aliphatischer, linearer oder verzweigter C₁-C₆-Kohlenwassrstoffrest ist,
- X, Y: unabhängig voneinander jeweils entfällt, -O-, -COO- oder -O-CO-NR³-, wobei R³ für H oder einen C₁-C₅-Alkylrest, vorzugsweise für H, Methyl und/oder Ethyl und ganz besonders bevorzugt für H steht, und wobei X und Y nicht gleichzeitig entfallen,
- Q²: entfällt, ein linearer oder verzweigter C₁-C₁₀-Alkylen-Rest, der durch ein oder mehrere O-Atome, vorzugsweise ein O-Atom, unterbrochen sein kann und der durch einen oder mehrere, vorzugsweise einen, Substituenten, die aus -OH und -OR⁴ ausgewählt sind, substituiert sein kann oder vorzugsweise unsubstituiert ist, wobei R⁴ ein aliphatischer, linearer oder verzweigter C₁-C₆-Kohlenwassrstoffrest ist,
- n: 1 oder 2, und wobei
die Substitution am Aromaten in 3-, vorzugsweise in 4-Stellung erfolgt.

Besonders bevorzugt haben die Variablen die folgenden Bedeutungen:
- Q¹: ein 1- oder 2-wertiger, aliphatischer, linearer oder verzweigter C₁-C₅-Kohlenwasserstoffrest, der durch ein O-Atom unterbrochen sein kann und der durch eine OH-Gruppe substituiert sein kann,
- X: -COO-,
- Y: entfällt,
- Q²: entfällt oder ein linearer C₁-C₃-Alkylen-Rest,
- n: 1 oder 2, und wobei die Substitution am Aromaten in 4-Stellung erfolgt.

Ganz besonders bevorzugt haben die Variablen die folgenden Bedeutungen:
- Q¹: ein 1- oder 2-wertiger, aliphatischer, verzweigter, vorzugsweise linearer C₁-C₄-Kohlenwasserstoffrest,
- X: -COO-,
- Y: entfällt,
- Q²: entfällt oder ein Methylen-Rest,
- n: 1 oder 2, und wobei die Substitution am Aromaten in 4-Stellung erfolgt.

Die für die einzelnen Variablen angegebenen bevorzugten, besonders bevorzugten und ganz besonders bevorzugten Definitionen können jeweils unabhängig voneinander ausgewählt werden. Verbindungen, in denen alle Variablen die bevorzugten, besonders bevorzugten und ganz besonders bevorzugten Definitionen aufweisen, sind naturgemäß erfindungsgemäß besonders geeignet.

Die Hydroperoxide der Formel I sind in Kombination mit einem Thioharnstoffderivat als Redoxinitiatorsystem wirksam. Erfindungsgemäß wurde überraschend gefunden, dass sie nicht den unangenehmen Geruch von Cumolhydroperoxid aufweisen.

Hydroperoxid-Derivate der Formel I lassen sich beispielsweise herstellen, indem substituierte Isopropylbenzolderivate mit einer terminalen OH-Gruppe mit einer geeigneten Carbonsäure verestert und anschließend durch Oxidation in erfindungsgemäße Hydroperoxid-Derivate der Formel I überführen werden:

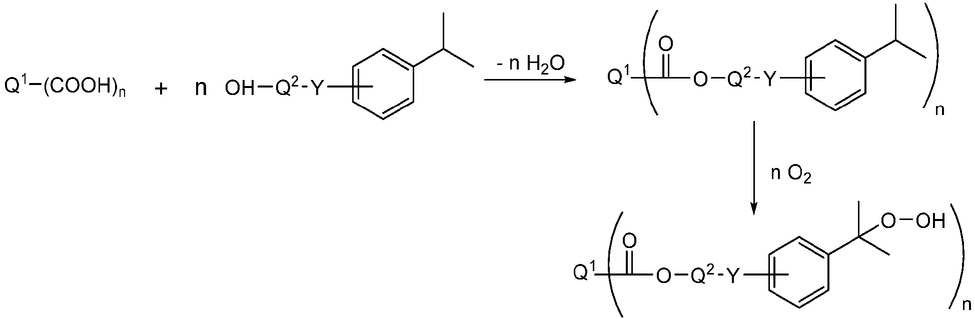

Ein konkretes Beispiel ist:

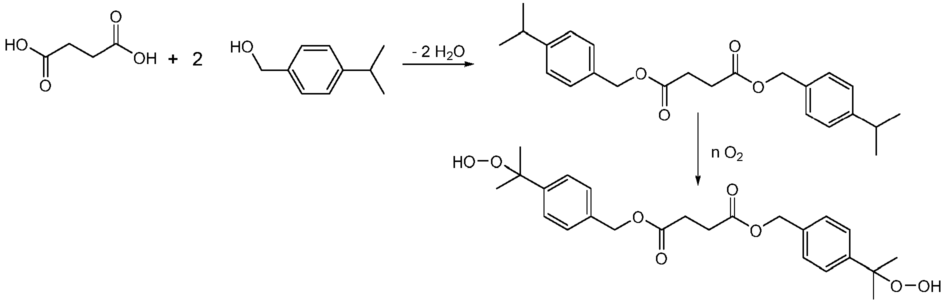

Erfindungsgemäß bevorzugte Hydroperoxid-Derivate der Formel I sind:

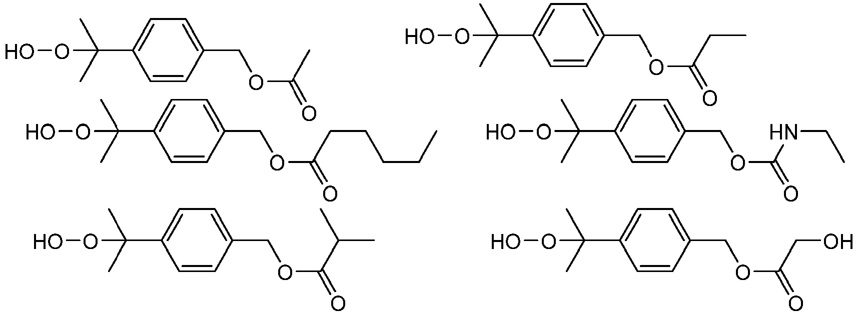

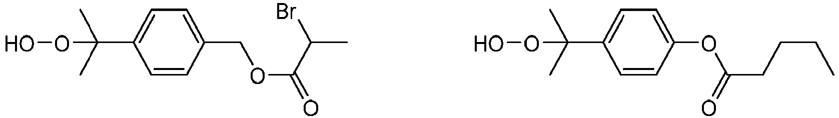

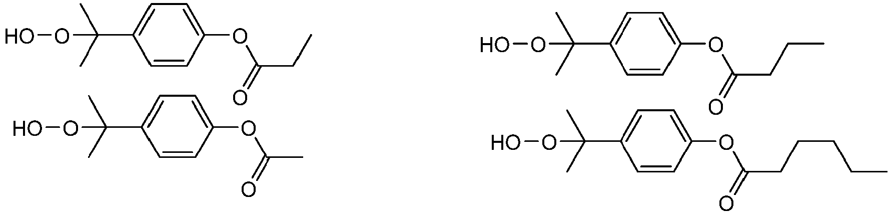

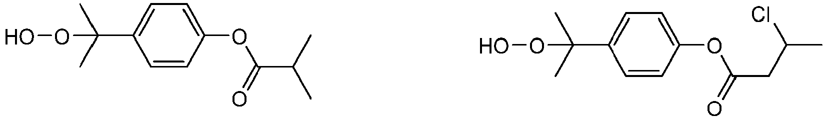

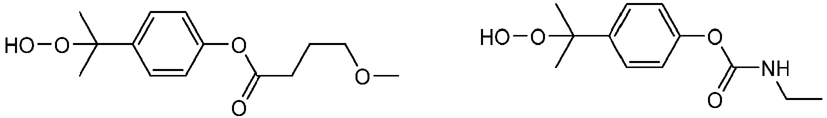

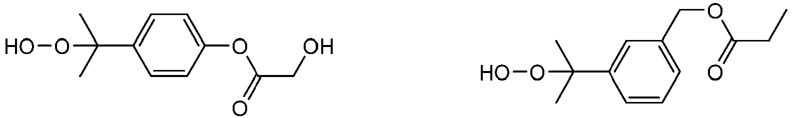

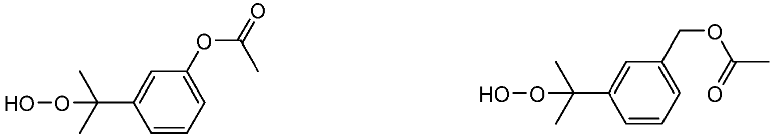

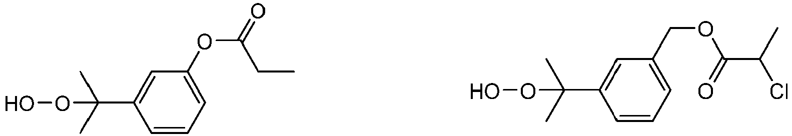

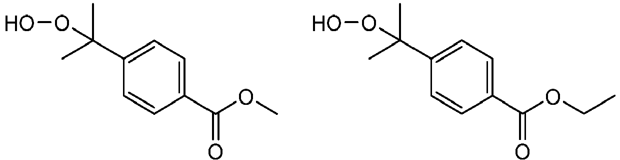

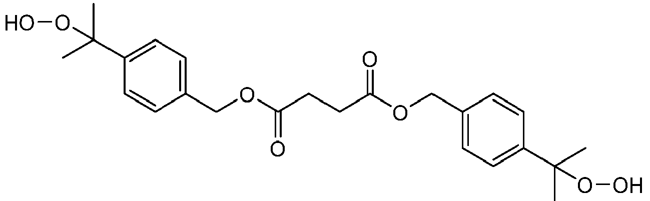

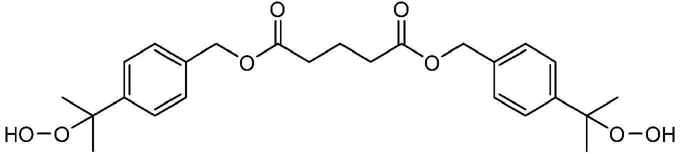

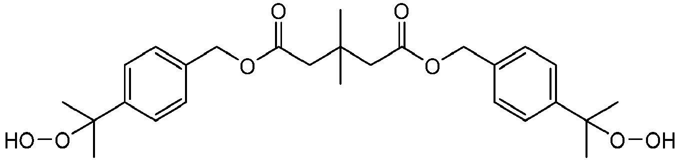

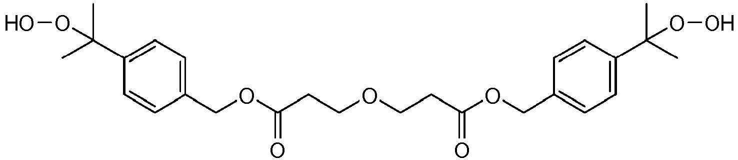

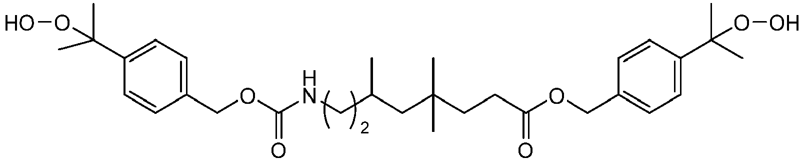

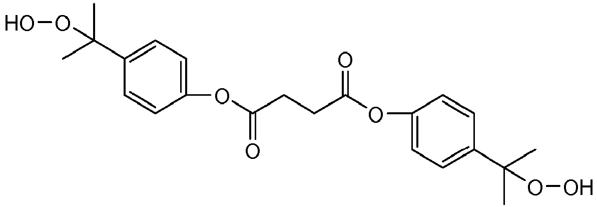

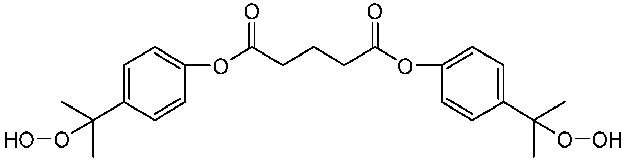

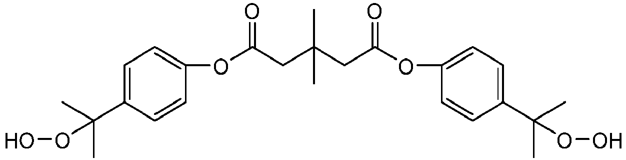

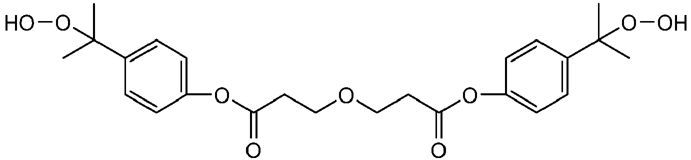

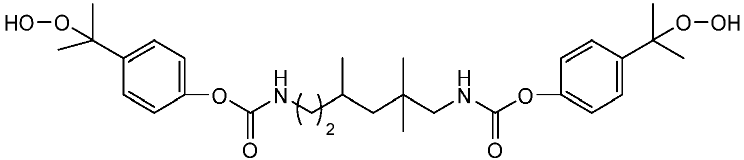

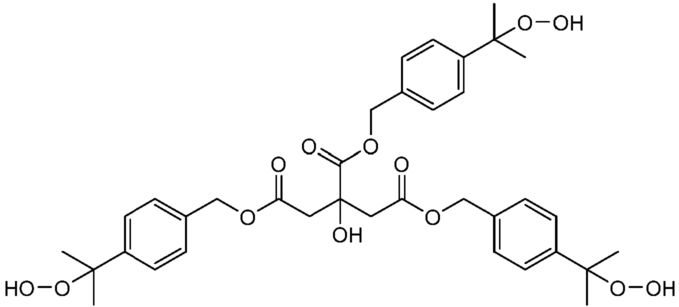

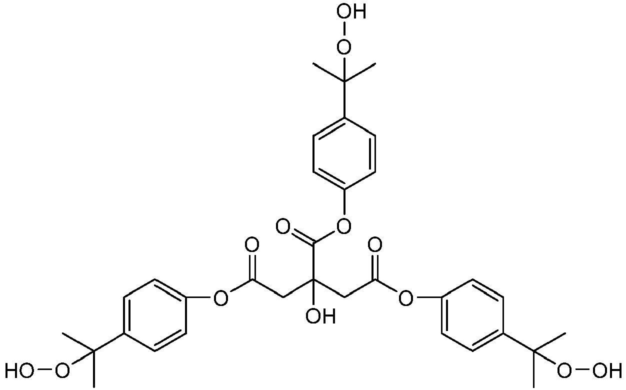

Die Hydroperoxid-Derivate der Formel I zeigen eine große Lagerstabilität bei Raumtemperatur und eignen sich besonders als geruchsarme Hydroperoxid-Komponente in Redoxinitiatorsystemen für dentale Zusammensetzungen. Die erfindungsgemäßen Werkstoffe können ein oder mehrere Hydroperoxide der Formel I enthalten.

Die erfindungsgemäßen Werkstoffe enthalten neben dem Hydroperoxid der Formel (I) mindestens ein Thioharnstoffderivat. Erfindungsgemäß bevorzugte Thioharnstoffderivate sind die in der EP 1 754 465 A1 in Absatz [0009] aufgeführten Verbindungen. Besonders bevorzugte Thioharnstoffderivate sind Acetyl-, Allyl-, Pyridyl- und Phenylthioharnstoff, Hexanoylthioharnstoff und Mischungen davon. Ganz besonders bevorzugt ist Acetylthioharnstoff (ATU).

Weiter bevorzugt sind Thioharnstoffderivate mit der Formel
in der 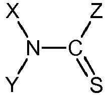
- X: H oder Y ist,
- Y: ein Alkylrest mit 1 bis 8 Kohlenstoffatomen, ein Cycloalkylrest mit 5 oder 6 Kohlenstoffatomen, ein Chlor-, Hydroxy- oder Mercapto-substituierter Alkylrest mit 1 bis 8 Kohlenstoffatomen, ein Alkenylrest mit 3 bis 4 Kohlenstoffatomen, ein Arylrest mit 6 bis 8 Kohlenstoffatomen, ein Chlor-, Hydroxy-, Methoxy- oder Sulfonyl-substituierter Phenylrest, ein Acylrest mit 2 bis 8 Kohlenstoffatomen, ein Chlor- oder Methoxy-substituierter Acylrest, ein Aralkylrest mit 7 bis 8 Kohlenstoffatomen oder ein Chlor- oder Methoxy-substituierter Aralkylrest ist, und
- Z: NH₂, NHX oder NX₂ ist.

Gemäß einer bevorzugten Ausführungsform enthalten die erfindungsgemäßen Dentalwerkstoffe neben dem Hydroperoxid der Formel I und dem Thioharnstoffderivat zusätzlich mindestens ein Peroxid. Es wurde überraschend gefunden, dass die Reaktivität des Initiatorsystems auf der Basis eines Hydroperoxids der Formel I und eines Thioharnstoffderivats durch den Zusatz einer geringer Mengen eines Peroxids deutlich beschleunigt werden kann.

Erfindungsgemäß bevorzugte Peroxid sind Verbindungen der Formel R⁵-(O-O-R⁶)ₘ, in der R⁵ und R⁶ jeweils für einen aliphatischen oder aromatischen Kohlenwasserstoffrest oder eine Acylgruppe stehen und m 1 oder 2 ist. Besonders bevorzugt sind Diacylperoxide. Bevorzugte aliphatische Kohlenwasserstoffreste sind Reste mit 3 bis 8 Kohlenstoffatomen, bevorzugte aromatische Kohlenwasserstoffreste sind Reste mit 6 bis 12 Kohlenstoffatomen, wobei Benzolreste, die mit 1 oder 2 Alkylgruppen substituiert sind, besonders bevorzugt sind. Bevorzugte Acylgruppen sind Gruppen, die 2 bis 20 Kohlenstoffatome enthalten.

Bevorzugte Peroxide in denen R⁵ und R⁶ jeweils für einen aliphatischen oder aromatischen Kohlenwasserstoffrest stehen sind a,a-Bis(t-butylperoxy)diisopropylbenzol, Dicumolperoxid, 2,5-Dimethyl-2,5-bis(t-butylperoxy)hexan, t-Butylcumylperoxid, di-t-Butylperoxid, 2,5-Dimethyl-2,5-bis(t-butylperoxy)hexin-3.

Bevorzugte Diacylperoxide sind Isobutyrylperoxid, 2,4-Dichlorbenzoylperoxid, 3,5,5-Trimethylhexanoylperoxid, Octanoylperoxid, Lauroylperoxid, Stearylperoxid, Bernsteinsäureperoxid, m-Toluoylbenzoylperoxid und Mischungen davon. Ein ganz besonders bevorzugtes Peroxid ist Benzoylperoxid (DBPO). Hydroperoxide sind keine Peroxide im Sinne der Erfindung.

Gemäß einer weiteren bevorzugten Ausführungsform enthalten die erfindungsgemäßen Dentalwerkstoffe neben dem Hydroperoxid der Formel I und dem Thioharnstoffderivat zusätzlich mindestens eine Übergangsmetallverbindung. Es wurde gefunden, dass die Zugabe einer Übergangsmetallverbindung Werkstoffe ergibt, die nach der Härtung erheblich verbesserte mechanische Eigenschaften aufweisen.

Erfindungsgemäß bevorzugte Übergangsmetallverbindungen sind Verbindungen, die sich von Übergangsmetallen ableiten, die mindestens zwei stabile Oxidationsstufen haben. Besonders bevorzugt sind Verbindungen der Elemente Kupfer, Eisen, Kobalt, Nickel und Mangan. Diese Metalle weisen die folgenden stabilen Oxydationsstufen auf: Cu(I)/Cu(II), Fe(II)/Fe(III), Co(II)/Co(III), Ni(II)/Ni(III), Mn(II)/Mn(III). Werkstoffe, die mindestens eine Kupfer-Verbindung enthalten, sind besonders bevorzugt.

Die Übergangsmetalle werden bevorzugt in Form ihrer Salze eingesetzt. Bevorzugte Salze sind die Nitrate, Acetate, 2-Ethyl-hexanoate und Halogenide, wobei Chloride besonders bevorzugt sind.

Die Übergangsmetalle können weiterhin vorteilhaft in komplexierter Form eingesetzt werden, wobei Komplexe mit chelatbildenden Liganden besonders bevorzugt sind. Bevorzugte einfache Liganden zur Komplexierung der Übergangsmetalle sind 2-Ethylhexanoat und THF. Bevorzugte chelatbildende Liganden sind 2-(2-Aminoethyl-amino)ethanol, aliphatische Amine, besonders bevorzugt 1,1,4,7,10,10-Hexamethyltriethylentetramin (HMTETA), N,N,N',N",N"-Pentamethyldiethylentriamin (PMDETA), Tris[2-(dimethylamino)ethyl]amin (Me₆TREN), N,N,N',N'-Tetramethylethylendiamin (TMEDA), 1,4,8,11-Tetraaza-1,4,8,11-tetramethylcyclotetradecan (Me4CYCLAM), Diethylentriamin (DETA), Triethylentetramin (TETA) und 1,4,8,11-Tetraazacyclotetradecan (CYCLAM)); pyridinhaltige Liganden, besonders bevorzugt N,N,N',N'-Tetrakis-(2-pyridylmethyl)ethylendiamin (TPEN), N,N-Bis(2-pyridylmethyl)amin (BPMA), N,N-Bis(2-pyridylmethyl)octylamin (BPMOA), 2,2'-Bipyridin und 8-Hydroxychinolin. Ganz besonders bevorzugte Liganden sind Acetylaceton, Dimethylglyoxim und 1,10-Phenanthrolin.

Bei elektrisch neutralen Liganden muss die Ladung der Übergangsmetallionen durch geeignete Gegenionen ausgeglichen werden. Hierzu kommen insbesondere die oben genannten Ionen in Betracht, die zur Bildung von Salzen verwendet werden, wobei Acetate und Chloride besonders bevorzugt sind. Chloride und Komplexe zeichnen sich durch eine relativ gute Löslichkeit in Monomeren aus, die zur Herstellung von Dentalwerkstoffen eingesetzt werden.

Anstelle der Übergangsmetallkomplexe können nichtkomplexe Salze der Übergangsmetalle in Kombination mit komplexbildenden organischen Verbindungen zur Herstellung der Dentalwerkstoffe eingesetzt werden, vorzugsweise in Kombination mit den oben genannten chelatbildenden Verbindungen. Die organischen Liganden bilden beim Mischen mit den Übergangsmetallsalzen die katalytisch wirksamen Komplexe. Die Verwendung solcher Kombinationen von Übergangsmetallsalzen und organischen Liganden ist bevorzugt.

Bevorzugt sind Übergangsmetallverbindungen der Metalle Kupfer, Eisen, Kobalt und Nickel.

Bevorzugte Kupfersalze sind CuCl, CuBr, CuCl₂, CuBr₂, CuI₂, Cu(II)-carboxylate (z.B. der Essigsäure oder 2-Ethylhexansäure). Bevorzugte Kupferkomplexe sind Komplexe mit den Liganden Acetylaceton, Phenanthrolin (z.B. 1,10-Phenanthrolin (Phen)), der aliphatischen Aminen, wie z.B. 1,1,4,7,10,10-Hexamethyltriethylentetramin (HMTETA), N,N,N',N",N"-Pentamethyldiethylentriamin (PMDETA), Tris[2-(dimethyl-amino)ethyl]amin (Me₆ TREN).

Bevorzugte Eisensalze sind FeCl₃, FeBr₂ und FeCl₂. Bevorzugte Eisenkomplexe sind Komplexe mit den Liganden Acetylaceton, Triphenylphosphin, 4,4'-Di(5-nonyl)-2,2'-bipyridin (dNbpy) oder 1,3-Diisopropyl-4,5-dimethylimidazol-2-yliden (Prilm). Ganz besonders bevorzugt sind die Komplexe Fe(acac)₂ und FeCl₂(PPh₃)₂.

Bevorzugte Nickelsalze sind NiBr₂ und NiCl₂, bevorzugte Nickelkomplexe sind Nickelacetylacetonat und NiBr₂(PPh₃)₂.

In allen Fällen sind solche Komplexe bevorzugt, in denen das jeweilige Übergangsmetall in seiner stabilsten Oxidationsstufe vorliegt. Bevorzugt sind damit Komplexe von Cu²⁺, Fe³⁺, Ni²⁺ und Co³⁺.

Erfindungsgemäß sind Kupferverbindungen, Kupferkomplexe und insbesondere Mischungen von Kupfersalzen und komplexierenden organischen Liganden besonders bevorzugt.

Ganz besonders bevorzugt sind Werkstoffe, die mindestens einem Hydroperoxid der Formel I, mindestens ein Thioharnstoffderivat, mindestens ein Peroxid und eine Übergangsmetallverbindung enthalten, wobei diese Komponenten vorzugsweise jeweils aus den oben definierten bevorzugten und besonders bevorzugten Stoffen ausgewählt sind.

Das Hydroperoxid wird vorzugsweise in einer Menge von 0,01 bis 10 Gew.-%, besonders bevorzugt 0,05 bis 8,0 Gew.-% und ganz besonders bevorzugt 0,1 bis 5,0 Gew.-% eingesetzt. Das Thioharnstoffderivat wird vorzugsweise in einer molaren Menge von 25 bis 100 mol-%, vorzugsweise 50 bis 100 mol-%, bezogen auf die molare Menge an Hydroperoxid, ganz besonders bevorzugt in der gleichen molaren Konzentration wie das Hydroperoxid eingesetzt.

Das Peroxid wird ggf. vorzugsweise in einer Menge von 1 bis 15 Gew.-%, bevorzugt 1 bis 10 Gew.-% und ganz besonders bevorzugt von 2 bis 8 Gew.-% eingesetzt, bezogen auf die Masse des Hydroperoxids.

Die Übergangsmetallverbindung wird ggf. vorzugsweise in einer Menge von 0,0001 bis 1 Gew.-%, bevorzugt 0,0005 bis 0,5 Gew.-% und besonders bevorzugt 0,0007 bis 0,020 Gew.-% eingesetzt, bezogen auf die Gesamtmasse der Zusammensetzung.

Hydroperoxide der Formel (I) eignen sich besonders zur Aushärtung radikalisch polymerisierbarer Zusammensetzungen.

Die erfindungsgemäßen Werkstoffe enthalten vorzugsweise mindestens ein radikalisch polymerisierbares Monomer. Besonders bevorzugt sind Zusammensetzungen, die als radikalisch polymerisierbares Monomer mindestens ein mono- oder multifunktionelles (Meth)acrylat enthalten. Unter monofunktionellen (Meth)acrylaten werden Verbindungen mit einer, unter multifunktionellen (Meth)¬acrylaten Verbindungen mit zwei oder mehr, vorzugsweise 2 bis 4 radikalisch polymerisierbaren Gruppen verstanden. Gemäß einer ganz besonders bevorzugten Ausführungsform enthalten die erfindungsgemäßen Zusammensetzungen mindestens ein Dimethacrylat oder eine Mischung von Mono- und Dimethacrylaten. Materialien, die intraoral gehärtet werden sollen, enthalten als radikalisch polymerisierbares Monomer vorzugsweise mono- und/oder multifunktionelle Methacrylate.

Bevorzugte mono- oder multifunktionelle (Meth)acrylate sind Methyl-, Ethyl-, 2-Hydroxyethyl-, Butyl-, Benzyl-, Tetrahydrofurfuryl- oder Isobornyl(meth)acrylat, p-Cumylphenoxyethylenglycolmethacrylat (CMP-1E), 2-(2-Biphenyloxy)-ethylmethacrylat, Bisphenol-A-dimethacrylat, Bis-GMA (ein Additionsprodukt aus Methacrylsäure und Bisphenol-A-diglycidylether), ethoxy- oder propoxyliertes Bisphenol-A-Dimethacrylat, wie z.B. 2-[4-(2-Methacryloyloxyethoxyethoxy)phenyl]-2-[4-(2-methacryloyloxyethoxy)phenyl]propan) (SR-348c, Firma Sartomer; enthält 3 Ethoxygruppen) und 2,2-Bis[4-(2-methacryloxypropoxy)phenyl]propan, UDMA (ein Additionsprodukt aus 2-Hydroxyethylmethacrylat und 2,2,4-Trimethylhexamethylen-1,6-diisocyanat), V-380 (ein Additionsprodukt aus einer Mischung von 0,7 mol 2-Hydroxyethylmethacrylat und 0,3 mol 2-Hydroxypropylmethacrylat mit 1 mol α,α,α',α'-Tetramethyl-m-xylylen-diisocyanat), Di-, Tri- oder Tetraethylenglycoldimethacrylat, Trimethylolpropantrimethacrylat, Pentaerythrittetramethacrylat sowie Glycerindi- und -trimethacrylat, 1,4-Butandioldimethacrylat, 1,10-Decandioldimethacrylat (D₃MA), Bis(methacryloyloxymethyl)tricyclo-[5.2.1.02,6]decan (DCP), Polyethylenglycol- oder Polypropylenglycoldimethacrylate, wie z.B. Polyethylenglycol-200- oder Polyethylenglycol-400-dimethacrylat (PEG-200- oder PEG-400-DMA) oder 1,12-Dodecandioldimethacrylat, oder eine Mischung davon.

Gemäß einer Ausführungsform enthaltend die erfindungsgemäßen Zusammensetzungen neben den oben genannten Monomeren vorzugsweise zusätzlich ein oder mehrere säuregruppenhaltige radikalisch polymerisierbare Monomere (Haftmonomere). Diese verleihen den Werkstoffen selbsthaftende und/oder selbstätzende Eigenschaften. Säuregruppenhaltige Monomere eignen sich daher besonders zur Herstellung von selbsthaftenden Dentalwerkstoffen, wie z.B. von Befestigungszementen.

Bevorzugte säuregruppenhaltige Monomere sind polymerisationsfähige Carbonsäuren, Phosphonsäuren und Phosphorsäureester sowie deren Anhydride. Bevorzugte Carbonsäuren und Carbonsäureanhydride sind 4-(Meth)acryloyloxyethyltrimellitsäureanhydrid, 10-Methacryloyloxydecylmalonsäure, N-(2-Hydroxy-3-methacryloyloxypropyl)-N-phenylglycin, 4-Vinylbenzoesäure. Bevorzugte Phosphorsäureester sind 2-Methacryloyloxyethyl-phenyl-hydrogenphosphat, 10-Methacryloyloxydecyldihydrogenphosphat (MDP) und Dipentaerythritpentamethacryloyloxyphosphat. Bevorzugte Phosphonsäuren sind 4-Vinylbenzylphosphonsäure, 2-[4-(Dihydroxyphosphoryl)-2-oxa-butyl]-acrylsäure und deren Amide, Ester, wie z.B. 2-[4-(Dihydroxyphosphoryl)-2-oxa-butyl]-acrylsäure-2,4,6-trimethylphenylester.

Besonders bevorzugte säuregruppenhaltige Monomere sind 4-Vinylbenzylphosphonsäure, 2-[4-(Dihydroxyphosphoryl)-2-oxa-butyl]-acrylsäure und deren Amide, Ester, wie z.B 2-[4-(Dihydroxyphosphoryl)-2-oxa-butyl]-acrylsäure-2,4,6-trimethylphenylester, (Meth)acrylamiddihydrogenphosphate, wie z.B. 6-Methacrylamidohexyl- oder 1,3-Bis(methacrylamido)-propan-2-yl-dihydrogenphosphat, und Mischungen davon. Diese besonders bevorzugten säuregruppenhaltigen Monomere zeichnen sich durch eine hohe Hydrolysestabilität aus.

Die erfindungsgemäßen Zusammensetzungen können neben dem erfindungsgemäßen Initiatorsystem vorteilhaft zusätzlich einen Initiator für die radikalische Photopolymerisation enthalten. Solche Zusammensetzungen sind dualhärtend, d.h. sie lassen sich sowohl chemisch als auch durch Licht härten. Bevorzugte Photoinitiatoren sind Benzophenon, Benzoin sowie deren Derivate, α-Diketone und deren Derivate, wie 9,10-Phenanthrenchinon, 1-Phenyl-propan-1,2-dion, Diacetyl und 4,4'-Dichlorbenzil. Bevorzugt werden Campherchinon (CC) und 2,2-Dimethoxy-2-phenylacetophenon in Kombination mit Aminen als Reduktionsmittel, wie z.B. 4-(Dimethyl-amino)-benzoesäureethylester (EDMAB), oder N,N-Dimethylaminoethylmethacrylat, verwendet.

Erfindungsgemäß sind solche Zusammensetzungen bevorzugt, die keine Amine enthalten. Besonders bevorzugt sind daher Norrish-Typ-I-Photoinitiatoren. Norrish-Typ-I-Photoinitiatoren benötigen keine Aminkomponente.

Bevorzugte Norrish-Typ-I-Photoinitiatoren sind Acyl- oder Bisacylphosphinoxide. Besonders bevorzugt sind Monoacyltrialkylgermanium-, Diacyldialkylgermanium- und Tetraacylgermanium-Verbindungen, wie z.B. Benzoyltrimethylgerman, Dibenzoyldiethylgerman, Bis(4-methoxybenzoyl)diethylgerman (Ivocerin®), Tetrabenzoylgerman und Tetrakis(o-methylbenzoyl)german.

Außerdem lassen sich auch Mischungen der verschiedenen Photoinitiatoren einsetzen, wie z.B. Bis(4-methoxybenzoyl)diethylgerman bzw. Tetrakis(o-methylbenzoyl)-german in Kombination mit Campherchinon und 4-Dimethylaminobenzoesäureethylester.

Die erfindungsgemäßen Dentalwerkstoffe können vorteilhaft außerdem einen oder mehrere organische oder anorganische Füllstoffe enthalten. Bevorzugt sind partikuläre Füllstoffe. Füllstoffhaltige Zusammensetzungen eignen sich besonders als dentale Befestigungszemente oder Füllungskomposite.

Bevorzugte anorganische Füllstoffe sind Oxide, wie SiO₂, ZrO₂ und TiO₂ oder Mischoxide aus SiO₂, ZrO₂, ZnO und/oder TiO₂, nanopartikuläre oder mikrofeine Füllstoffe, wie pyrogene Kieselsäure oder Fällungskieselsäure, Glaspulver, wie Quarz-, Glaskeramik-, Borosilikat- oder röntgenopake Glaspulver, vorzugsweise Barium- oder Strontiumaluminiumsilikatgläser, und röntgenopake Füllstoffe, wie Ytterbiumtrifluorid, Tantal(V)-oxid, Bariumsulfat oder Mischoxide von SiO₂ mit Ytterbium(III)-oxid oder Tantal(V)-oxid. Weiterhin können die erfindungsgemäßen Dentalwerkstoffe faserförmige Füllstoffe, Nanofasern, Whiskers oder Mischungen davon enthalten.

Bevorzugt weisen die Oxide eine Partikelgröße von 0,010 bis 15 µm, die nanopartikulären oder mikrofeinen Füllstoffe eine Partikelgröße von 10 bis 300 nm, die Glaspulver eine Partikelgröße von 0,01 bis 15 µm, vorzugweise von 0,2 bis 1,5 µm, und die röntgenopaken Füllstoffe eine Partikelgröße von 0,2 bis 5 µm auf.

Besonders bevorzugte Füllstoffe sind Mischoxide aus SiO₂ und ZrO₂, mit einer Partikelgröße von 10 bis 300 nm, Glaspulver mit einer Partikelgröße von 0,2 bis 1,5 µm, insbesondere röntgenopake Glaspulver von z.B. Barium- oder Strontiumaluminiumsilikatgläsern, und röntgenopake Füllstoffe mit einer Partikelgröße von 0,2 bis 5 µm, insbesondere Ytterbiumtrifluorid und/oder Mischoxide von SiO₂ mit Ytterbium(III)-oxid.

Außerdem sind als Füllstoff gemahlene Präpolymerisate oder Perlpolymerisate (Isofüller) geeignet. Diese können ausschließliche aus organischen Polymeren oder aus organischen Polymeren bestehen, die ihrerseits mit anorganischen Füllstoffen wie röntgenopakem/n Glaspulver(n) und Ytterbiumtrifluorid gefüllt sind. Zur Herstellung der gemahlenen Präpolymerisate und Perlpolymere eignen sich die oben definierten Monomere und Füllstoffe. Zusammensetzungen zur Herstellung dentaler Totalprothesen enthalten als Füllstoffe vorzugsweise ausschließlich organische Füllstoffe, besonders bevorzugt gemahlene Polymerisate oder Perlpolymerisate auf Basis von Polymethylmethacrylat (PMMA), ganz besonders bevorzugt Perlpolymerisate auf der Basis von PMMA.

Wenn nicht anders angegeben, handelt es sich bei allen Partikelgrößen um gewichtsmittlere Partikelgrößen, wobei die Partikelgrößenbestimmung im Bereich von 0,1 µm bis 1000 µm mittels statischer Lichtstreuung erfolgt, vorzugsweise mit einem statischen Laserstreuungs-Partikelgrößen-Analysator LA-960 (Horiba, Japan). Hierbei werden als Lichtquellen eine Laser-Diode mit einer Wellenlänge von 655 nm und eine LED mit einer Wellenlänge von 405 nm verwendet. Der Einsatz von zwei Lichtquellen mit unterschiedlichen Wellenlängen ermöglicht die Vermessung der gesamten Partikelgrößenverteilung einer Probe in nur einem Messungsdurchgang, wobei die Messung als Nassmessung durchgeführt wird. Hierzu wird eine 0,1 bis 0,5%ige wässrige Dispersion des Füllstoffs hergestellt und deren Streulicht in einer Durchflusszelle gemessen. Die Streulichtanalyse zur Berechnung von Partikelgröße und Partikelgrößenverteilung erfolgt gemäß der Mie-Theorie nach DIN/ISO 13320.

Partikelgrößen kleiner als 0,1 µm werden vorzugsweise mittels Dynamischer Lichtstreuung (DLS) bestimmt. Die Messung der Partikelgröße im Bereich von 5 nm bis 0,1 µm erfolgt vorzugsweise durch Dynamische Lichtstreuung (DLS) von wässrigen Partikeldispersionen, vorzugsweise mit einem Malvern Zetasizer Nano ZS (Malvern Instruments, Malvern UK) mit einem He-Ne-Laser mit einer Wellenlänge von 633 nm, bei einem Streuwinkel von 90° bei 25°C.

Partikelgrößen kleiner als 0,1 µm können auch mittels REM- oder TEM-Spektroskopie bestimmt werden. Die Transmissionselektronenmikroskopie (TEM) wird vorzugsweise mit einem Philips CM30 TEM bei einer Beschleunigungsspannung von 300 kV durchgeführt. Für die Probenvorbereitung werden Tropfen der Partikeldispersion auf einem 50 Å dickem Kupfergitter (Maschenweite 300) aufgebracht, das mit Kohlenstoff beschichtet ist, und im Anschluss das Lösungsmittel verdampft.

Die Lichtstreuung nimmt mit abnehmender Partikelgröße ab, jedoch haben Füllstoffe mit geringer Partikelgröße eine größere Verdickungswirkung. Die Füllstoffe werden nach ihrer Partikelgröße unterteilt in Makrofüller und Mikrofüller, wobei Füllstoffe mit einer mittleren Partikelgröße von 0,2 bis 10 µm als Makrofüller und Füllstoffe mit einer mittlere Partikelgröße von ca. 5 bis 100 nm als Mikrofüller bezeichnet werden. Makrofüller werden z.B. durch Mahlen z.B. von Quarz, röntgenopaken Gläsern, Borosilikaten oder von Keramik gewonnen und bestehen meist aus splitterförmigen Teilen. Mikrofüller wie Mischoxide können z.B. durch hydrolytische Co-Kondensation von Metallalkoxiden hergestellt werden.

Zur Verbesserung des Verbundes zwischen den Füllstoffpartikeln und der vernetzten Polymerisationsmatrix sind die Füllstoffe vorzugsweise oberflächenmodifiziert, besonders bevorzugt durch Silanisierung, ganz besonders bevorzugt durch radikalisch polymerisierbare Silane, insbesondere mit 3-Methacryloyloxypropyltrimethoxysilan. Zur Oberflächenmodifizierung von nicht-silikatischen Füllstoffen, z.B. von ZrO₂ oder TiO₂, können auch funktionalisierte saure Phosphate, wie z.B. 10-Methacryloyloxydecyldihydrogenphosphat eingesetzt werden.

Außerdem können die erfindungsgemäßen Dentalwerkstoffe ein oder mehrere weitere Additive enthalten, vor allem Stabilisatoren, Farbmittel, mikrobiozide Wirkstoffe, fluoridionenabgebende Additive, Treibmittel, optische Aufheller, Weichmacher und/oder UV-Absorber.

Erfindungsgemäß sind Dentalwerkstoffe bevorzugt, die
(a) 0,01 bis 10 Gew.-%, bevorzugt 0,05 bis 8,0 Gew.-% und besonders bevorzugt 0,1 bis 3,0 Gew.-% Hydroperoxid der Formel (I),
(b) 0,001 bis 5,0 Gew.-%, bevorzugt 0,003 bis 4,0 Gew.-%, besonders bevorzugt 0,005 bis 3,0 Gew.-% Thioharnstoff und/oder Thioharnstoffderivat,
(c) 5 bis 95 Gew.-%, bevorzugt 10 bis 95 Gew.-% und besonders bevorzugt 10 bis 90 Gew.-% radikalisch polymerisierbares Monomer,
(d) 0 bis 85 Gew.-% Füllstoff, und
(e) 0,01 bis 5 Gew.-%, bevorzugt 0,1 bis 3 Gew.-% und besonders bevorzugt 0,1 bis 2 Gew.-% Additiv enthalten.

Alle Mengenangaben hierin beziehen sich auf die Gesamtmasse der Zusammensetzung, wenn nicht anders angegeben.

Der Füllungsgrad richtet sich nach dem gewünschten Anwendungszweck des Werkstoffs. Füllungskomposite haben vorzugsweise einen Füllstoffgehalt von 50 bis 85 Gew.-%, besonders bevorzugt 70 bis 80 Gew.-%, und dentale Zemente von 10 bis 70 Gew.-%, besonders bevorzugt 60 bis 70 Gew.-%.

Besonders bevorzugt sind Dentalwerkstoffe, die zusätzlich
(f) 0,001 bis 3,0 Gew.-%, bevorzugt 0,005 bis 2,0 Gew.-% und besonders bevorzugt 0,005 bis 0,50 Gew.-% Peroxid, vorzugsweise DBPO
   und /oder
(g) 0,0001 bis 1 Gew.-%, bevorzugt 0,0005 bis 0,5 Gew.-%, besonders bevorzugt 0,0007 bis 0,02 Gew.-% Übergangsmetallverbindung enthalten.

Dentalwerkstoffe zur Herstellung von Totalprothesen weisen vorzugsweise die folgende Zusammensetzung auf:
(a) 0,01 bis 5,0 Gew.-%, bevorzugt 0,05 bis 4,0 Gew.-% und besonders bevorzugt 0,1 bis 3,0 Gew.-% Hydroperoxid der Formel (I),
(b) 0,001 bis 5,0 Gew.-%, besonders bevorzugt 0,005 bis 2,0 Gew.-% Thioharnstoff und/oder Thioharnstoff-Derivat,
(c) 20 bis 95 Gew.-%, bevorzugt 30 bis 95 Gew.-% und besonders bevorzugt 40 bis 95 Gew.-% radikalisch polymerisierbares Monomer,
(d) 5 bis 80 Gew.-%, bevorzugt 10 bis 70 Gew.-% und bevorzugt besonders bevorzugt 20 bis 60 Gew.-% Isofüller,
(e) 0,01 bis 5 Gew.-%, bevorzugt 0,1 bis 3 Gew.-% und besonders bevorzugt 0,1 bis 2 Gew.-% Additiv.

Besonders bevorzugt sind solche Dentalwerkstoffe, die aus den genannten Komponenten bestehen, wobei die einzelnen Komponenten vorzugsweise jeweils aus den oben genannten bevorzugten und besonders bevorzugten Stoffen ausgewählt sind. In allen Fällen kann als jeweilige Komponente auch eine Mischung von mehreren Stoffen eingesetzt werden, also beispielsweise eine Mischung von Monomeren.

Die erfindungsgemäßen Zusammensetzungen eignen sich besonders als Dentalwerkstoffe, insbesondere als dentale Zemente, Füllungskomposite und Verblendmaterialien sowie als Materialien zur Herstellung von Prothesen, künstlichen Zähnen, Inlays, Onlays, Kronen und Brücken. Die Zusammensetzungen eignen sich primär zur intraoralen Anwendung durch den Zahnarzt zur Restauration geschädigter Zähne, d.h. zur therapeutischen Anwendung, z.B. als dentale Zemente, Füllungskomposite und Verblendmaterialien. Sie können aber auch nicht-therapeutisch (extraoral) eingesetzt werden, beispielsweise bei der Herstellung oder Reparatur von Dentalrestaurationen, wie Prothesen, künstlichen Zähnen, Inlays, Onlays, Kronen und Brücken.

Die erfindungsgemäßen Zusammensetzungen eignen sich außerdem zur Herstellung von Formkörpern für dentale aber auch für nicht dentale Zwecke, die z.B. mittels Gießen, Pressen und insbesondere durch generative Verfahren wie den 3D Druck hergestellt werden können.

Die Erfindung wird im Folgenden anhand von Ausführungsbeispielen näher erläutert:

### Ausführungsbeispiele

### Beispiel 1

### Synthese von 4-(2-Hydroperoxypropan-2-yl)-phenylpropionat

### 1. Stufe: (4-Isopropylphenyl)-propionat (IPPP)

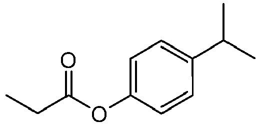

Zu einer Lösung von 523.0 g (3.84 mol) 4-Isopropylphenol, 388.6 g (3.84 mol) Triethylamin und 46.9 g (0.38 mol) 4-Dimethylaminopyridin in 4.50 L Dichlormethan wurden bei 0°C 499.7 g (3.84 mol) Propionsäureanhydrid zugetropft. Das Gemisch wurde langsam auf Raumtemperatur erwärmen gelassen. Nach insgesamt 15 h Reaktionszeit wurde die Lösung wie folgt gewaschen: zweimal mit je 1.50 L 1N Salzsäure, zweimal mit je 1.50 L 1N Natronlauge sowie zweimal mit je 1.50 L Wasser. Nach dem Trocknen der organischen Phase mit wasserfreiem Natriumsulfat wurde das Lösungsmittel komplett abdestilliert. Es wurden 699.8 g (95%) IPPP als klare, farblose Flüssigkeit in einer Reinheit von 98.82% (HPLC) erhalten.
***n_{D}²⁰***: 1.4916
**¹H NMR** (400 MHz, CDCl₃): δ (ppm) = 1.23 [d, *J* = 7.0 Hz, 6H, HC(CH₃)₂], 1.25 (t, *J* = 7.6 Hz, 3H, CH₂CH₃), 2.55 (t, *J* = 7.6 Hz, 2H, CH₂CH₃), 2.89 [sept., *J* = 7.0 Hz, 1H, HC(CH₃)₂], 6.97-7.00 und 7.19-7.23 (2 m, je 2H, =CH).
**¹³C NMR** (100 MHz, CDCl₃): δ (ppm) = 9.13 (CH₂CH₃), 24.1 [HC(CH₃)₂], 27.8 (CH₂CH₃), 33.7 [HC(CH₃)₂], 121.3 und 127.3 (=CH), 146.2 und 148.8 (=C), 173.1 (C=O).
**IR** (diamond ATR): v (cm⁻¹) = 2964 (m, C-H), 1758 (s, C=O), 1720 (m, C=C), 1604 und 1508 (m, s, Aromat), 1462 (m, CH₂, CH₃), 1352 (m, CH₃), 1200 und 1148 (vs, COC), 836 (m, =CH).

### 2. Stufe: 4-(2-Hydroperoxypropan-2-yl)-phenylpropionat(IPPPHP)

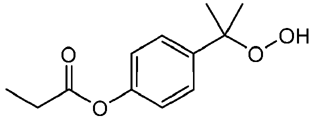

33.3 g (0.173 mol) IPPP und 2.82 g (0.017 mol) *N*-Hydroxyphthalimid wurden in 600 mL Acetonitril aufgelöst, auf 30°C erwärmt und für 10 min ein Sauerstoffstrom durch die Lösung geleitet. Nach der Zugabe von 2.60 g (0.009 mol) 2,2'-Azobis-(4-methoxy-2,4-dimethylvaleronitril) wurde das Reaktionsgemisch 5 Tage unter Sauerstoffatmosphäre bei 30°C gerührt. Nach dem Entfernen des Lösungsmittels erfolgte eine chromatographische Reinigung des Rohproduktes über Kieselgel (n-Heptan/Ethylacetat 4:1). 8.90 g (23%) **IPPPHP** wurden als klares, farbloses, nahezu geruchloses Öl mit einem Hydroperoxid-Gehalt von 85% (Titration) erhalten.
***n_{D}²⁰***: 1.5100
**¹H NMR** (400 MHz, CDCl₃): δ (ppm) = 1.25 (t, *J* = 7.5 Hz, 3H, CH₂CH₃), 1.57 (s, 6H, CH₃), 2.58 (t, *J* = 7.5 Hz, 2H, CH₂CH₃), 7.04-7.07 und 7.43-7.47 (2 m, je 2H, =CH), 7.75 (s, 1H, OOH).
**¹³C NMR** (100 MHz, CDCl₃): δ (ppm) = 9.06 (CH₂CH₃), 26.1 [C(CH₃)₂], 27.7 (CH₂CH₃), 83.5 [C(CH₃)₂], 121.4 und 126.7 (=CH), 142.2 und 149.9 (=C), 173.2 (C=O).
IR (Diamond ATR): v (cm⁻¹) = 3416 (m, OH), 2983 (m, C-H), 1758 (s, C=O), 1725 (s, C=C), 1605 und 1507 (m, s, Aromat), 1462 (m, CH₂, CH₃), 1360 (m, CH₃), 1201 und 1148 (vs, COC), 834 (m, =CH).

### Beispiel 2

### 4-(2-Hydroperoxypropan-2-yl)-benzyl-(4-isopropylbenzyl)-succinat (HPS)

### 1. Stufe: Synthese von Bis-(4-isopropylbenzyl)-succinat

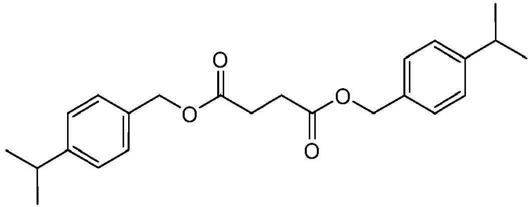

Zu einer Lösung von 78.4 g (0.522 mol) 4-Isopropylbenzylalkohol, 30.8 g (0.261 mol) Bernsteinsäure und 1.59 g (0.013 mol) DMAP in 0.35 L Dichlormethan wurde bei 0°C eine Lösung von 61.9 g (0.30 mol) *N*,*N*'-Dicyclohexylcarbodiimid (DCC) in 250 mL Dichlormethan getropft. Das Reaktionsgemisch wurde 18 h bei Raumtemperatur gerührt, anschliessend mit weiteren 1.60 g (0.013 mol) DMAP und 8.00 g (0.039 mol) DCC versetzt und für weitere 5 Tage bei Raumtemperatur gerührt. Nach dem Abfiltrieren der festen Bestandteile wurde das Filtrat 3x mit je 150 mL 2N Salzsäure, 3x mit je 150 mL 2N Natronlauge sowie 3x mit je 150 mL gesättigter NatriumchloridLösung gewaschen, mit wasserfreiem Natriumsulfat getrocknet und eingeengt. Die weitere Reinigung erfolgte chromatographisch über Kieselgel mit *n-*Heptan/Ethylacetat (3:1) als Eluent. Nach dem Einengen wurden 40.5 g (41%) Bis-(4-isopropylbenzyl)-succinat als klare, farblose Flüssigkeit erhalten.
***n_{D}²⁰***: 1.5249
**¹H NMR** (400 MHz, CDCl₃): δ (ppm) = 1.23 [d, *J* = 6.9 Hz, 12H, HC(CH₃)₂], 2.68 (s, 4H, O=C-CH₂), 2.90 [sept., *J* = 6.9 Hz, 2H, HC(CH₃)₂], 5.08 (s, 4H, OCH₂), 7.20-7.27 (m, 8H, =CH).
**¹³C** NMR (100 MHz, CDCl₃): δ (ppm) = 24.0 [HC(CH₃)₂], 29.2 (O=C-CH₂), 33.9 [HC(CH₃)₂], 66.6 (OCH₂), 126.7 und 128.5 (=CH), 133.2 (=C-CH₂O), 149.1 (=C-CH), 172.1 (C=O).
IR (diamond ATR): v (cm⁻¹) = 2960 (m, C-H), 1733 (vs, C=O), 1615 und 1515 (w, m, Aromat), 1463 (m, CH₂, CH₃), 1349 (m, CH₃), 1149 (vs, COC), 817 (s, =CH).

### 2. Stufe: Synthese von 4-(2-Hydroperoxypropan-2-yl)-benzyl-(4-isopropylbenzyl)-succinat (HPS)

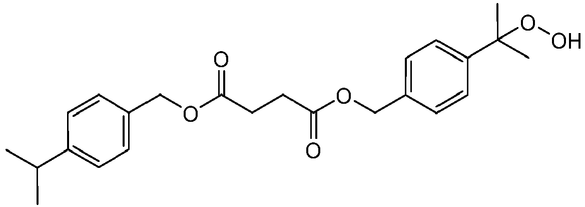

20.0 g (52.3 mmol) Bis-(4-isopropylbenzyl)-succinat und 0.85 g (5.23 mmol) *N*-Hydroxyphthalimid wurden in 300 mL Acetonitril aufgelöst, auf 30°C erwärmt und für 10 min ein Sauerstoffstrom durch die Lösung geleitet. Nach der Zugabe von 0.78 g (2.61 mmol) 2,2'-Azobis-(4-methoxy-2,4-dimethylvaleronitril) wurde das Reaktionsgemisch 6 Tage unter Sauerstoffatmosphäre bei 30°C gerührt. Nach dem Entfernen des Lösungsmittels wurde der Rückstand in 15 mL Dichlormethan aufgenommen und vom ausgefallenen Feststoff abfiltriert. Nach dem Entfernen des Dichlormethans erfolgte eine chromatographische Reinigung des Rohproduktes über Kieselgel (n-Heptan/Ethylacetat 3:1). 3.34 g (14%) **HPS** wurden als farbloses, fast geruchsloses Öl mit einem Hydroperoxid-Gehalt von 95.5% (Titration) erhalten.
***n_{D}²⁰***: 1.5314
**¹H NMR** (400 MHz, CDCl₃): δ (ppm) = 1.24 [d, *J* = 7.0 Hz, 6H, HC(CH₃)₂], 1.59 (s, 6H, OC(CH₃)₂], 2.69 (s, 4H, O=C-CH₂), 2.90 [sept., *J* = 6.9 Hz, 1H, HC(CH₃)₂], 5.08 und 5.10 (2 s, je 2H, OCH₂), 7.20-7.27 und 7.33-7.46 (2 m, je 4H, =CH).
**¹³C NMR** (100 MHz, CDCl₃): δ (ppm) = 23.9 [HC(CH₃)₂], 26.1 [OC(CH₃)₂], 29.2 (O=C-CH₂), 33.9 [HC(CH₃)₂], 66.2 und 66.6 (OCH₂), 83.8 [OC(CH₃)₂], 125.7, 126.6, 128.4 und 128.5 (=CH), 133.2 und 134.9 (=C-CH₂O), 144.9 (=C-CO), 149.1 (=C-CH), 172.1 und 172.2 (C=O).
**IR** (diamond ATR): v (cm⁻¹) = 3415 (br, OH), 2961 (m, C-H), 1733 (vs, C=O), 1610 und 1515 (w, m, Aromat), 1462 (m, CH₂, CH₃), 1350 (m, CH₃), 1152 (vs, COC), 818 (s, =CH).

### Beispiel 3

### Kompositzement auf der Basis des Hydroperoxids IPPPHP aus Beispiel 1

Durch Mischen der Dimethacrylate UDMA (Additionsprodukt aus 2-Hydroxyethylmethacrylat und 2,2,4-Trimethylhexamethylen-1,6-diisocyanat), V-380 (ein Additionsprodukt aus einer Mischung von 0,7 mol 2-Hydroxyethylmethacrylat und 0,3 mol 2-Hydroxypropylmethacrylat mit 1 mol α,α,α',α'-Tetramethyl-m-xylylen-diisocyanat), DCP (Bis(methacryloyloxymethyl)tricyclo-[5.2.1.0^{2,6}]decan) und PEG-400-DMA (Polyethylenglycol-400-dimethacrylat), dem Stabilisator BHT (2,6-Di-tert.-butyl-4-methylphenol), den pyrogenen Kieselsäuren HDK 2000 (Wacker) Aerosil OX-50 (Evonik) sowie den Initiatorkomponenten Cumolhydroperoxid (Merk, CHP) bzw. IPPPHP, Kupfer(II)-acetylacetonat (Cuacac) und Hexanoylthioharnstoff (HTH) wurden chemisch härtende Kompositzemente jeweils bestehend aus einer Basenpaste und einer Katalysatorpaste gemäß Tabelle 1 hergestellt.

**Tabelle 1: Zusammensetzung der Katalysatorpasten Kat-1 und Kat-2 und der Basenpasten Base-1 und Base-2 (Angaben in Gew.-%)**

| **Komponente** | **Kat-1** | **Kat-2** | **Base-1** | **Base-2** |
|---|---|---|---|---|
| UDMA | 19,75 | 19,99 | 20,93 | 20,90 |
| V-380 | 11,05 | 11,16 | 11,41 | 11,39 |
| DCP | 6,89 | 6,99 | 7,12 | 7,13 |
| PEG-400-DMA | 8,27 | 8,37 | 8,54 | 8,54 |
| HDK 2000 | 3,50 | 3,50 | 3,50 | 3,50 |
| Aerosil OX 50 | 47,99 | 48,01 | 47,96 | 48,01 |
| BHT | 0,05 | 0,05 | 0,10 | 0,10 |
| CHP (80 %-ig) | 2,50 | - | - | - |
| IPPPHP (8 %-ig) | - | 1,94 | - | - |
| HTA | - | - | 0,49 | 0,49 |
| Cuacac | - | - | 0,03 | 0,03 |

Die Pasten Kat-1 und Bas-1 bzw. Kat-2 und Base-2 wurden jeweils im Volumenverhältnis 1:1 mit einer Doppelschubspritze mit Mischkanüle vermischt und die Verarbeitungszeit und die mechanischen Eigenschaften der erhaltenen Zemente C-1 und C-2 bestimmt. Die Bestimmung der Verarbeitungszeit und der mechanischen Eigenschaften erfolgte nach der Norm EN ISO-4049 (Dentistry - Polymer-based filling, restorative and luting materials). Zur Bestimmung der Verarbeitungszeit VZ wurden die Pasten unmittelbar nach dem Mischen in das Proberöhrchen einer Exotherm-Einrichtung mit Thermoelement (Thermoelement Typ K (Thermocoax FKI 10/50NN); Fa. Hersteller: THERMOCONTROL GmbH, Dietikon/Schweiz) eingefüllt, wobei die Zeitmessung mit Mischbeginn startete. Der Beginn der Aushärtung ist mit einer Temperaturerhöhung verbunden, welche die Exotherm-Einrichtung mit einem Kurvenanstieg anzeigt. Der Zeitpunkt des Temperaturanstieges entspricht dem Beginn der Härtungsreaktion und damit das Ende der Verarbeitungszeit. Die Verarbeitungszeit ist die Zeitphase von Mischbeginn bis zum Aushärtungsbeginn. Zur Bestimmung der mechanischen Eigenschaften wurden Prüfkörper hergestellt und deren Biegefestigkeit BF und Biege-E-Modul nach der Norm EN ISO-4049 ermittelt. Die Messung der mechanischen Eigenschaften erfolgte nach 24 h Lagerung der Prüfkörper in Wasser (WL) bei 37°C. Die Ergebnisse sind in Tabelle 2 angegeben (Tabelle 2).

**Tabelle 2: Verarbeitungszeit (VZ, s), Biegefestigkeit (BF, MPa) und Biege-E-Modul (BM, GPa) der Zemente C-1 und C-2**

| **Zement** | **VZ** | **BF** | **Biege-E-Modul** |
|---|---|---|---|
| C-1 (Kat-1+Base-1)*) | 49 | 143 | 6,02 |
| C-2 (Kat-2+Base-2) | 57 | 123 | 5,58 |

| | | | |
|---|---|---|---|
| *) Vergleichsbeispiel | | | |

Die Ergebnisse belegen, dass der Zement C-2 auf der Basis des nahezu geruchsfreien Cumolhydroperoxids IPPPHP aus Beispiel 1 vergleichbare Eigenschaften zeigt, wie der Zement C1 auf des Basis des sehr stark riechenden Cumolhydroperoxids CHP.

### Beispiel 4

### Kompositzement auf der Basis des Hydroperoxids HPS aus Beispiel 2

Aus einer Mischung der Dimethacrylate UDMA, SR-348c (ethoxyliertes Bisphenol-A-dimethacrylat Bisphenol-A-dimethacrylat mit 3 Ethoxygruppen) und DCP, dem Stabilisator BHT (2,6-Di-tert.-butyl-4-methylphenol), den pyrogenen Kieselsäuren HDK 200 (Wacker) und SG-So100 (Sukgyung) sowie den Initiatorkomponenten HPS (aus Beispiel 2), Kupfer(I)-Chlorid (CuCI), Hexanoylthioharnstoff (HTH) und 2-Mercaptobenzimidazol (2-MBI) wurde ein chemisch härtender Kompositzement mit der Base-Paste Base-3 und der Katalysatorpaste Kat-3 hergestellt (Tabelle 3).

**Tabelle 3: Zusammensetzung der Katalysatorpaste Kat-3 und der Basenpaste Base-3 (Angaben in Gew.-%)**

| **Komponente** | **Kat-3** | **Base-3** |
|---|---|---|
| UDMA | 17,84 | 19,475 |
| SR-348C | 22,31 | 23,73 |
| DCP | 4,47 | 4,75 |
| HDK 2000 | 3,02 | 3,0 |
| SG-Sol 100 | 48,00 | 48,00 |
| BHT | 0,05 | 0,05 |
| HPS (92 %-ig) | 4,31 | - |
| 2-MBI | - | 0,49 |
| HTH | - | 0,49 |
| CuCl | - | 0,015 |

Analog zu Beispiel 3 wurden Prüfkörper hergestellt und die Verarbeitungszeit VZ und die mechanischen Eigenschaften bestimmt (Tabelle 4).

**Tabelle 4: Verarbeitungszeit (VZ, s), Biegefestigkeit (BF, MPa) und Biege-E-Modul (BM, GPa) des Zements C-3**

| **Zement** | **VZ** | **BF** | **Biege-E-Modul** |
|---|---|---|---|
| C-3 (Kat-3+Base-3) | 35 | 103 | 3,96 |

## Patentansprüche

1. Radikalisch polymerisierbarer Dentalwerkstoff, der als Initiatorsystem für die radikalische Polymerisation eine Kombination aus einem Thioharnstoffderivat und einem Hydroperoxid enthält, **dadurch gekennzeichnet, dass** er ein Hydroperoxid gemäß der folgenden Formel (I) enthält, 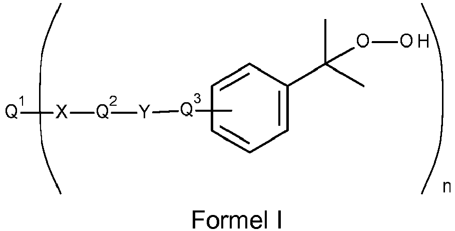 in der die Variablen die folgenden Bedeutungen haben:
Q¹ ein n-wertiger, aromatischer, aliphatischer, linearer oder verzweigter C₁-C₁₄-Kohlenwasserstoffrest, der durch ein oder mehrere S- und/oder O-Atome unterbrochen sein kann und der unsubstituiert oder durch einen oder mehrere Substituenten, die vorzugsweise aus -OH, -OR¹, -Cl und-Br ausgewählt sind, substituiert sein kann, wobei R¹ ein aliphatischer, linearer oder verzweigter C₁-C₁₀-Kohlenwassrstoffrest ist,
X, Y unabhängig voneinander jeweils entfällt, -O-, -COO-; -CONR²-, oder -O-CO-NR³-, wobei R² und R³ unabhängig voneinander für H oder einen C₁-C₅-Alkylrest, vorzugsweise für H, Methyl und/oder Ethyl, besonders bevorzugt H stehen, und wobei X und Y nicht gleichzeitig entfallen,
Q² entfällt, ein aliphatischer, linearer oder verzweigter C₁-C₁₄-Alkylen-Rest, der durch S- und/oder O-Atome unterbrochen sein kann und der unsubstituiert oder durch -OH, -OR⁴, -Cl und/oder -Br substituiert sein kann, wobei R⁴ ein aliphatischer, linearer oder verzweigter C₁-C₁₀-Kohlenwasserstoffrest ist,
Q³ eine C₁-C₃-Alkylengruppe oder entfällt, vorzugsweise -CH₂- oder entfällt,
wobei X oder Y entfällt, wenn Q² entfällt,
n 1, 2, 3 oder 4, und wobei
die Substitution am Aromaten in 2-, 3- oder 4-Stellung erfolgt.

2. Dentalwerkstoff nach Anspruch 1, bei dem die Variablen die folgenden Bedeutungen haben:
Q¹ ein 1- oder 2-wertiger, aliphatischer, linearer oder verzweigter C₁-C₁₀-Kohlenwasserstoffrest, der durch ein oder mehrere O-Atome, vorzugsweise ein O-Atom, unterbrochen sein kann und der durch einen oder mehrere, vorzugsweise einen, Substituenten, die aus -OH und -OR¹ ausgewählt sind, substituiert sein kann oder vorzugsweise unsubstituiert ist, wobei R¹ ein aliphatischer, linearer oder verzweigter C₁-C₆-Kohlenwassrstoffrest ist,
X, Y unabhängig voneinander jeweils entfällt, -O-, -COO- oder -O-CO-NR³-, wobei R³ für H oder einen C₁-C₅-Alkylrest, vorzugsweise für H, Methyl und/oder Ethyl und ganz besonders bevorzugt für H steht, und wobei X und Y nicht gleichzeitig entfallen,
Q² entfällt, ein linearer oder verzweigter C₁-C₁₀-Alkylen-Rest, der durch ein oder mehrere O-Atome, vorzugsweise ein O-Atom, unterbrochen sein kann und der durch einen oder mehrere, vorzugsweise einen, Substituenten, die aus -OH und -OR⁴ ausgewählt sind, substituiert sein kann oder vorzugsweise unsubstituiert ist, wobei R⁴ ein aliphatischer, linearer oder verzweigter C₁-C₆-Kohlenwassrstoffrest ist,
n 1 oder 2, und wobei
die Substitution am Aromaten in 3-, vorzugsweise in 4-Stellung erfolgt.

3. Dentalwerkstoff nach Anspruch 2, bei dem die Variablen die folgenden Bedeutungen haben:
Q¹ ein 1- oder 2-wertiger, aliphatischer, linearer oder verzweigter C₁-C₅-Kohlenwasserstoffrest, der durch ein O-Atom unterbrochen sein kann und der durch eine OH-Gruppe substituiert sein kann,
X -COO-,
Y entfällt,
Q² entfällt oder ein linearer C₁-C₃-Alkylen-Rest,
n 1 oder 2, und wobei
die Substitution am Aromaten in 4-Stellung erfolgt.

4. Dentalwerkstoff nach Anspruch 3, bei dem die Variablen die folgenden Bedeutungen haben:
Q¹ ein 1- oder 2-wertiger, aliphatischer, verzweigter, vorzugsweise linearer C₁-C₄ -Kohlenwasserstoffrest,
X -COO-,
Y entfällt,
Q² entfällt oder ein Methylen-Rest,
n 1 oder 2, und wobei
die Substitution am Aromaten in 4-Stellung erfolgt.

5. Dentalwerkstoff nach einem der vorangehenden Ansprüche, der als Thioharnstoffderivat Acetyl-, Allyl-, Pyridyl-, Phenylthioharnstoff, Hexanoylthioharnstoff oder eine Mischung davon enthält, vorzugsweise Acetylthioharnstoff (ATU).

6. Dentalwerkstoff nach einem der vorangehenden Ansprüche, der zusätzlich ein Peroxid enthält, vorzugsweise α,α-Bis(t-butylperoxy)diisopropylbenzol, Dicumolperoxid, 2,5-Dimethyl-2,5-bis(t-butylperoxy)hexan, t-Butylcumylperoxid, di-t-Butylperoxid, 2,5-Dimethyl-2,5-bis(t-butylperoxy)hexin-3 oder eine Mischung davon, besonders bevorzugt ein Diacylperoxid wie Isobutyrylperoxid, 2,4-Dichlorbenzoylperoxid, 3,5,5-Trimethylhexanoylperoxid, Octanoylperoxid, Lauroylperoxid, Stearylperoxid, Bernsteinsäureperoxid, m-Toluoylbenzoylperoxid oder eine Mischung davon, ganz besonders bevorzugt Benzoylperoxid (DBPO).

7. Dentalwerkstoff nach einem der vorangehenden Ansprüche, der zusätzlich eine Übergangsmetallverbindung enthält, vorzugsweise eine Verbindung eines Übergangsmetalls, das mindestens zwei stabile Oxidationsstufen hat, vorzugsweise eine Verbindungen des Kupfers, Eisens, Kobalts, Nickels, Mangans oder eine Mischung davon.

8. Dentalwerkstoff nach einem der vorangehenden Ansprüche, der
- 0,01 bis 10 Gew.-%, bevorzugt 0,05 bis 8,0 Gew.-% und besonders bevorzugt 0,1 bis 5,0 Gew.-% Hydroperoxid, bezogen auf die Gesamtmasse des Werkstoffs,
- 25 bis 100 mol-%, bevorzugt 50 bis 100 mol-% und besonders bevorzugt eine äquimolare Menge Thioharnstoffderivat, bezogen auf die molare Menge an Hydroperoxid,
- ggf. 1 bis 15 Gew.-%, bevorzugt 1 bis 10 Gew.-% und besonders bevorzugt 2 bis 8 Gew.-% Peroxid, bezogen auf die Masse des Hydroperoxids, und
- ggf. 0,0001 bis 1 Gew.-%, bevorzugt 0,0005 bis 0,5 Gew.-% und besonders bevorzugt 0,0007 bis 0,020 Gew.-% Übergangsmetallverbindung enthält, bezogen auf die Gesamtmasse der Zusammensetzung.

9. Dentalwerkstoff nach einem der vorangehenden Ansprüche, der zusätzlich mindestens ein radikalisch polymerisierbares Monomer enthält, vorzugsweise mindestens ein mono- oder multifunktionelles (Meth)acrylat, besonders bevorzugt mindestens ein Dimethacrylat oder eine Mischung von Mono- und Dimethacrylaten.

10. Dentalwerkstoff nach Anspruch 9, der als radikalisch polymerisierbares Monomer Methyl-, Ethyl-, 2-Hydroxyethyl-, Butyl-, Benzyl-, Tetrahydrofurfuryl- oder Isobornyl(meth)acrylat, p-Cumyl-phenoxyethylenglycolmethacrylat (CMP-1E), 2-(2-Biphenyloxy)-ethylmethacrylat, Bisphenol-A-dimethacrylat, Bis-GMA (ein Additionsprodukt aus Methacrylsäure und Bisphenol-A-diglycidylether), ethoxy- oder propoxyliertes Bisphenol-A-Dimethacrylat, 2-[4-(2-Methacryloyloxyethoxyethoxy)phenyl]-2-[4-(2-methacryloyloxyethoxy)phenyl]propan) (SR-348c), 2,2-Bis[4-(2-methacryloxypropoxy)phenyl]propan, UDMA (ein Additionsprodukt aus 2-Hydroxyethylmethacrylat und 2,2,4-Trimethylhexamethylen-1,6-diisocyanat), V-380 (ein Additionsprodukt aus einer Mischung von 0,7 mol 2-Hydroxyethylmethacrylat und 0,3 mol 2-Hydroxypropylmethacrylat mit 1 mol α,α,α',α'-Tetramethyl-m-xylylen-diisocyanat), Di-, Tri- oder Tetraethylenglycoldimethacrylat, Trimethylolpropantrimethacrylat, Pentaerythrittetramethacrylat, Glycerindi- und -trimethacrylat, 1,4-Butandioldimethacrylat, 1,10-Decandioldimethacrylat (D₃MA), Bis(methacryloyloxymethyl)tricyclo-[5.2.1.02,6]decan (DCP), ein Polyethylenglycol- oder Polypropylenglycoldimethacrylat, Polyethylenglycol-200- ,Polyethylenglycol-400-dimethacrylat (PEG-200- oder PEG-400-DMA), 1,12-Dodecandioldimethacrylat oder eine Mischung davon enthält.

11. Dentalwerkstoff nach Anspruch 9 oder 10, der zusätzlich mindestens ein säuregruppenhaltiges radikalisch polymerisierbares Monomer enthält, vorzugsweise eine polymerisationsfähige Carbonsäure, Phosphonsäure, einen polymerisationsfähigen Phosphorsäureester oder ein Anhydrid dieser Stoffe.

12. Dentalwerkstoff nach einem der vorangehenden Ansprüche, der zusätzlich mindestens einen organischen oder anorganischen Füllstoff enthält, vorzugsweise ein Oxid, wie SiO₂, ZrO₂ und TiO₂ oder ein Mischoxid aus SiO₂, ZrO₂, ZnO und/oder TiO₂, einen nanopartikulären oder mikrofeinen Füllstoff, wie pyrogene Kieselsäure oder Fällungskieselsäure, Glaspulver, wie Quarz-, Glaskeramik- oder röntgenopakes Glaspulver, vorzugsweise Barium- oder Strontiumaluminiumsilikatglaspulver, einen röntgenopaken Füllstoff, wie Ytterbiumtrifluorid, Tantal(V)-oxid, Bariumsulfat, ein Mischoxid von SiO₂ mit Ytterbium(III)-oxid oder Tantal(V)-oxid, ein gemahlenes Präpolymerisat oder ein Perlpolymerisat.

13. Dentalwerkstoff nach einem der vorangehenden Ansprüche, der
(a) 0,01 bis 10 Gew.-%, bevorzugt 0,05 bis 8,0 Gew.-% und besonders bevorzugt 0,1 bis 3,0 Gew.-% Hydroperoxid der Formel (I),
(b) 0,001 bis 5,0 Gew.-%, bevorzugt 0,003 bis 4,0 Gew.-%, besonders bevorzugt 0,005 bis 3,0 Gew.-% Thioharnstoff und/oder Thioharnstoffderivat,
(c) 5 bis 95 Gew.-%, bevorzugt 10 bis 95 Gew.-% und besonders bevorzugt 10 bis 90 Gew.-% radikalisch polymerisierbares Monomer,
(d) 0 bis 85 Gew.-% Füllstoff, und
(e) 0,01 bis 5 Gew.-%, bevorzugt 0,1 bis 3 Gew.-% und besonders bevorzugt 0,1 bis 2 Gew.-% Additiv enthält,
jeweils bezogen auf die Gesamtmasse des Werkstoffs.

14. Dentalwerkstoff nach Anspruch 13, der 50 bis 85 Gew.-% oder 10 bis 70 Gew.-% Füllstoff enthält.

15. Dentalwerkstoff nach einem der vorangehenden Ansprüche zur therapeutischen Anwendung, vorzugsweise als dentaler Zement, Füllungskomposit oder Verblendmaterial.

16. Nicht-therapeutische Verwendung eines Dentalwerkstoffs gemäß einem der Ansprüche 1 bis 13, zur Herstellung oder Reparatur von Dentalrestaurationen, wie Prothesen, künstlichen Zähnen, Inlays, Onlays, Kronen, Brücken und Totalprothesen.

## Claims

1. Radically polymerizable dental material, which comprises a combination of a thiourea derivative and a hydroperoxide as initiator system for the radical polymerization, **characterized in that** it comprises a hydroperoxide according to the following Formula (I), 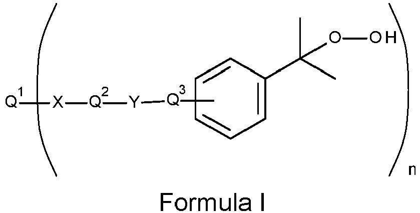 in which the variables have the following meanings:
Q¹ an n-valent, aromatic, aliphatic, linear or branched C₁-C₁₄ hydrocarbon radical, which can be interrupted by one or more S and/or O atoms and which can be unsubstituted or substituted by one or more substituents which are preferably selected from -OH, -OR¹, -Cl and -Br, wherein R¹ is an aliphatic, linear or branched C₁-C₁₀ hydrocarbon radical,
X, Y independently of each other are in each case absent, -O-, -COO-; -CONR²-, or -O-CO-NR³-, wherein R² and R³ independently of each other represent H or a C₁-C₅ alkyl radical, preferably H, methyl and/or ethyl, more preferably H, and wherein X and Y are not absent at the same time,
Q² is absent, an aliphatic, linear or branched C₁-C₁₄ alkylene radical, which can be interrupted by S and/or O atoms and which can be unsubstituted or substituted by -OH, -OR⁴, -Cl and/or -Br, wherein R⁴ is an aliphatic, linear or branched C₁-C₁₀ hydrocarbon radical,
Q³ a C₁-C₃ alkylene group or is absent, preferably -CH₂- or is absent, wherein X or Y is absent if Q² is absent,
n 1, 2, 3 or 4, and wherein
the substitution on the aromatic ring takes place in position 2, 3 or 4.

2. Dental material according to claim 1, in which the variables have the following meanings:
Q¹ a mono- or divalent, aliphatic, linear or branched C₁-C₁₀ hydrocarbon radical, which can be interrupted by one or more O atoms, preferably one O atom, and which can be substituted by one or more, preferably one, substituents which are selected from -OH and -OR¹, or is preferably unsubstituted, wherein R¹ is an aliphatic, linear or branched C₁-C₆ hydrocarbon radical,
X, Y independently of each other are in each case absent, -O-, -COO- or -O-CO-NR³-, wherein R³ represents H or a C₁-C₅ alkyl radical, preferably H, methyl and/or ethyl and more preferably H, and wherein X and Y are not absent at the same time,
Q² is absent, a linear or branched C₁-C₁₀ alkylene radical, which can be interrupted by one or more O atoms, preferably one O atom, and which can be substituted by one or more, preferably one, substituents which are selected from -OH and -OR⁴, or is preferably unsubstituted, wherein R⁴ is an aliphatic, linear or branched C₁-C₆ hydrocarbon radical,
n 1 or 2, and wherein
the substitution on the aromatic ring takes place in position 3, preferably in position 4.

3. Dental material according to claim 2, in which the variables have the following meanings:
Q¹ a mono- or divalent, aliphatic, linear or branched C₁-C₅ hydrocarbon radical, which can be interrupted by one O atom and which can be substituted by one OH group,
X -COO-,
Y is absent,
Q² is absent or a linear C₁-C₃ alkylene radical,
n 1 or 2, and wherein
the substitution on the aromatic ring takes place in position 4.

4. Dental material according to claim 3, in which the variables have the following meanings:
Q¹ a mono- or divalent, aliphatic, branched, preferably linear C₁-C₄ hydrocarbon radical,
X -COO-,
Y is absent
Q² is absent or a methylene radical,
n 1 or 2, and wherein
the substitution on the aromatic ring takes place in position 4.

5. Dental material according to any one of the preceding claims, which comprises acetyl thiourea, allyl thiourea, pyridyl thiourea, phenyl thiourea, hexanoyl thiourea or a mixture thereof, preferably acetyl thiourea (ATU), as thiourea derivative.

6. Dental material according to any one of the preceding claims, which additionally comprises a peroxide, preferably α,α-bis(t-butylperoxy)diisopropylbenzene, dicumene peroxide, 2,5-dimethyl-2,5-bis(t-butylperoxy)hexane, t-butyl cumyl peroxide, di-t-butyl peroxide, 2,5-dimethyl-2,5-bis(t-butylperoxy)hexyne-3 or a mixture thereof, more preferably a diacyl peroxide such as isobutyryl peroxide, 2,4-dichlorobenzoyl peroxide, 3,5,5-trimethylhexanoyl peroxide, octanoyl peroxide, lauroyl peroxide, stearyl peroxide, succinic acid peroxide, m-toluoyl benzoyl peroxide or a mixture thereof, most preferably benzoyl peroxide (DBPO).

7. Dental material according to any one of the preceding claims, which additionally comprises a transition metal compound, preferably a compound of a transition metal which has at least two stable oxidation states, preferably a compound of copper, iron, cobalt, nickel, manganese or a mixture thereof.

8. Dental material according to any one of the preceding claims, which comprises
- 0.01 to 10 wt.-%, preferably 0.05 to 8.0 wt.-% and more preferably 0.1 to 5.0 wt.-% hydroperoxide, relative to the total mass of the material,
- 25 to 100 mol-%, preferably 50 to 100 mol-% and more preferably an equimolar quantity of thiourea derivative, relative to the molar quantity of hydroperoxide,
- optionally 1 to 15 wt.-%, preferably 1 to 10 wt.-% and more preferably 2 to 8 wt.-% peroxide, relative to the mass of the hydroperoxide, and
- optionally 0.0001 to 1 wt.-%, preferably 0.0005 to 0.5 wt.-% and more preferably 0.0007 to 0.020 wt.-% transition metal compound, relative to the total mass of the composition.

9. Dental material according to any one of the preceding claims, which additionally comprises at least one radically polymerizable monomer, preferably at least one mono- or multifunctional (meth)acrylate, more preferably at least one dimethacrylate or a mixture of mono- and dimethacrylates.

10. Dental material according to claim 9, which comprises methyl, ethyl, 2-hydroxyethyl, butyl, benzyl, tetrahydrofurfuryl or isobornyl (meth)acrylate, p-cumylphenoxyethylene glycol methacrylate (CMP-1E), 2-(2-biphenyloxy)ethyl methacrylate, bisphenol A dimethacrylate, bis-GMA (an addition product of methacrylic acid and bisphenol A diglycidyl ether), ethoxylated or propoxylated bisphenol A dimethacrylate, 2-[4-(2-methacryloyloxyethoxyethoxy)phenyl]-2-[4-(2-methacryloyloxyethoxy)phenyl]propane) (SR-348c), 2,2-bis[4-(2-methacryloxypropoxy)phenyl]propane, UDMA (an addition product of 2-hydroxyethyl methacrylate and 2,2,4-trimethylhexamethylene-1,6-diisocyanate), V-380 (an addition product of a mixture of 0.7 mol 2-hydroxyethyl methacrylate and 0.3 mol 2-hydroxypropyl methacrylate with 1 mol α,α,α',α'-tetramethyl-m-xylylene diisocyanate), di-, tri- or tetraethylene glycol dimethacrylate, trimethylolpropane trimethacrylate, pentaerythritol tetramethacrylate, glycerol di- and trimethacrylate, 1,4-butanediol dimethacrylate, 1,10-decanediol dimethacrylate (D₃MA), bis(methacryloyloxymethyl)tricyclo-[5.2.1.02,6]decane (DCP), a polyethylene glycol or polypropylene glycol dimethacrylate, polyethylene glycol 200 dimethacrylate, polyethylene glycol 400 dimethacrylate (PEG 200 DMA or PEG 400 DMA), 1,12-dodecanediol dimethacrylate or a mixture thereof as radically polymerizable monomer.

11. Dental material according to claim 9 or 10, which additionally comprises at least one acid-group-containing radically polymerizable monomer, preferably a polymerizable carboxylic acid, phosphonic acid, a polymerizable phosphoric acid ester or an anhydride of these substances.

12. Dental material according to any one of the preceding claims, which additionally comprises at least one organic or inorganic filler, preferably an oxide, such as SiO₂, ZrO₂ and TiO₂ or a mixed oxide of SiO₂, ZrO₂, ZnO and/or TiO₂, a nanoparticulate or microfine filler, such as pyrogenic silica or precipitated silica, glass powder, such as quartz, glass ceramic or radiopaque glass powder, preferably barium or strontium aluminium silicate glass powder, a radiopaque filler, such as ytterbium trifluoride, tantalum(V) oxide, barium sulfate, a mixed oxide of SiO₂ with ytterbium(III) oxide or tantalum(V) oxide, a ground prepolymer or a pearl polymer.

13. Dental material according to any one of the preceding claims, which comprises
(a) 0.01 to 10 wt.-%, preferably 0.05 to 8.0 wt.-% and more preferably 0.1 to 3.0 wt.-% hydroperoxide of Formula (I),
(b) 0.001 to 5.0 wt.-%, preferably 0.003 to 4.0 wt.-%, more preferably 0.005 to 3.0 wt.-% thiourea and/or thiourea derivative,
(c) 5 to 95 wt.-%, preferably 10 to 95 wt.-% and more preferably 10 to 90 wt.-% radically polymerizable monomer,
(d) 0 to 85 wt.-% filler, and
(e) 0.01 to 5 wt.-%, preferably 0.1 to 3 wt.-% and more preferably 0.1 to 2 wt.-% additive,
in each case relative to the total mass of the material.

14. Dental material according to claim 13, which comprises 50 to 85 wt.-% or 10 to 70 wt.-% filler.

15. Dental material according to any one of the preceding claims for therapeutic application, preferably as dental cement, filling composite or veneering material.

16. Non-therapeutic use of a dental material according to any one of claims 1 to 13, for the production or repair of dental restorations, such as prostheses, artificial teeth, inlays, onlays, crowns, bridges and complete dentures.

## Revendications

1. Matériau dentaire polymérisable par voie radicalaire, qui contient en tant que système amorceur pour la polymérisation radicalaire une association d'un dérivé de thiourée et d'un hydroperoxyde, **caractérisé en ce qu'**il contient un hydroperoxyde selon la formule (I) suivante, 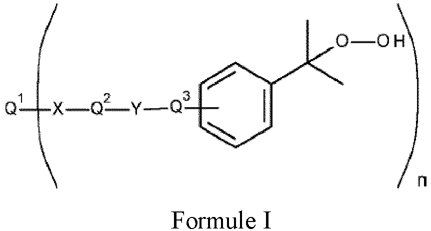 dans laquelle les variables ont les significations suivantes :
Q¹ représente un radical hydrocarboné en C₁-C₁₄ n-valent, aromatique, aliphatique, linéaire ou ramifié, qui peut être interrompu par un ou plusieurs atome(s) d'oxygène et/ou de soufre et qui peut être non substitué ou porteur d'un ou de plusieurs substituant(s) qui est/sont choisi(s) de préférence parmi -OH, -OR¹, -Cl et -Br, R¹ étant un radical hydrocarboné en C₁-C₁₀ aliphatique, linéaire ou ramifié,
X, Y indépendamment l'un de l'autre, sont chacun absents ou représentent chacun -O-, -COO- ; -CONR²-, ou -O-CO-NR³-, R² et R³ représentant indépendamment l'un de l'autre un atome d'hydrogène ou un radical alkyle en C₁-C₅, de préférence un atome d'hydrogène ou un groupe méthyle et/ou un groupe éthyle, de façon particulièrement préférée un atome d'hydrogène, et X et Y n'étant pas absents simultanément,
Q² est absent ou représente un radical alkylène en C₁-C₁₄ aliphatique, linéaire ou ramifié, qui peut être interrompu par des atomes d'oxygène et/ou de soufre et qui peut être non substitué ou substitué par -OH, -OR⁴, -Cl et/ou -Br, R⁴ étant un radical hydrocarboné en C₁-C₁₀ aliphatique, linéaire ou ramifié,
Q³ représente un groupe alkylène en C₁-C₃ ou est absent, de préférence est un groupe -CH₂- ou est absent,
X ou Y étant absent lorsque Q² est absent,
n vaut 1, 2, 3 ou 4, et
la substitution sur le cycle aromatique s'effectuant en position 2, 3 ou 4.

2. Matériau dentaire selon la revendication 1, dans lequel les variables ont les significations suivantes :
Q¹ représente un radical hydrocarboné en C₁-C₁₀ mono- ou divalent, aliphatique, linéaire ou ramifié, qui peut être interrompu par un ou plusieurs atome(s) d'oxygène, de préférence un atome d'oxygène et qui peut être porteur d'un ou de plusieurs, de préférence d'un, substituant(s) qui est/sont choisi(s) parmi -OH et -OR¹, R¹ étant un radical hydrocarboné en C₁-C₆ aliphatique, linéaire ou ramifié,
X, Y indépendamment l'un de l'autre, sont chacun absents ou représentent chacun -O-, -COO- ou -O-CO-NR³-, R³ représentant un atome d'hydrogène ou un radical alkyle en C₁-C₅, de préférence un atome d'hydrogène ou un groupe méthyle et/ou un groupe éthyle, et de façon tout particulièrement préférée un atome d'hydrogène, et X et Y n'étant pas absents simultanément,
Q² est absent ou représente un radical alkylène en C₁-C₁₀ linéaire ou ramifié, qui peut être interrompu par un ou plusieurs atome(s) d'oxygène, de préférence un atome d'oxygène et qui peut être substitué par un ou plusieurs, de préférence un, substituant(s) qui est/sont choisi(s) parmi -OH et -OR⁴, ou de préférence est non substitué, R⁴ étant un radical hydrocarboné en C₁-C₆ aliphatique, linéaire ou ramifié,
n vaut 1 ou 2, et
la substitution sur le cycle aromatique s'effectuant en position 3, de préférence en position 4.

3. Matériau dentaire selon la revendication 2, dans lequel les variables ont les significations suivantes :
Q¹ représente un radical hydrocarboné en C₁-C₅ mono- ou divalent, aliphatique, linéaire ou ramifié, qui peut être interrompu par un atome d'oxygène et qui peut être substitué par un groupe OH,
X est un groupe -COO-,
Y est absent,
Q² est absent ou est un radical alkylène en C₁-C₃ linéaire,
n vaut 1 ou 2, et
la substitution sur le cycle aromatique s'effectuant en position 4.

4. Matériau dentaire selon la revendication 3, dans lequel les variables ont les significations suivantes :
Q¹ représente un radical hydrocarboné en C₁-C₄ mono- ou divalent, aliphatique, ramifié, de préférence linéaire,
X est un groupe -COO-,
Y est absent,
Q² est absent ou est un radical méthylène,
n vaut 1 ou 2, et
la substitution sur le cycle aromatique s'effectuant en position 4.

5. Matériau dentaire selon l'une quelconque des revendications précédentes, qui contient, en tant que dérivé de thiourée, de l'acétyl-, allyl-, pyridyl-, phénylthiourée, de l'hexanoylthiourée ou un mélange de telles thiourées, de préférence de l'acétylthiourée (ATU).

6. Matériau dentaire selon l'une quelconque des revendications précédentes, qui contient en plus un peroxyde, de préférence de l'α,α-bis(tert-butylperoxy)diisopropylbenzène, du peroxyde de dicumène, du 2,5-diméthyl-2,5-bis(tert-butylperoxy)hexane, du peroxyde de tert-butylcumyle, du peroxyde de di-tert-butyle, du 2,5-diméthyl-2,5-bis(tert-butylperoxy)hexyne-3 ou mélange de tels peroxydes, de façon particulièrement préférée un peroxyde de diacyle tel que le peroxyde d'isobutyryle, le peroxyde de 2,4-dichlorobenzoyle, le peroxyde de 3,5,5-triméthylhexanoyle, le peroxyde d'octanoyle, le peroxyde de lauroyle, le peroxyde de stéaryle, le peroxyde d'acide succinique, le peroxyde de m-toluoylbenzoyle ou un mélange de tels peroxydes, de façon tout particulièrement préférée du peroxyde de benzoyle (DBPO).

7. Matériau dentaire selon l'une quelconque des revendications précédentes, qui contient en outre un composé de métal de transition, de préférence un composé d'un métal de transition qui a au moins deux degrés d'oxydation stables, de préférence un composé du cuivre, fer, cobalt, nickel, manganèse ou un mélange de ceux-ci.

8. Matériau dentaire selon l'une quelconque des revendications précédentes, qui contient
- 0,01 à 10 % en poids, de préférence 0,05 à 8,0 % en poids et de façon particulièrement préférée 0,1 à 5,0 % en poids d'hydroperoxyde, par rapport à la masse totale du matériau,
- 25 à 100 % en moles, de préférence 50 à 100 % en moles et de façon particulièrement préférée une quantité équimolaire de dérivé de thiourée, par rapport à la quantité molaire d'hydroperoxyde,
- éventuellement 1 à 15 % en poids, de préférence 1 à 10 % en poids et de façon particulièrement préférée 2 à 8 % en poids de peroxyde, par rapport à la masse de l'hydroperoxyde, et
- éventuellement 0,0001 à 1 % en poids, de préférence 0,0005 à 0,5 % en poids et de façon particulièrement préférée 0,0007 à 0,020 % en poids de composé de métal de transition, par rapport à la masse totale de la composition,

9. Matériau dentaire selon l'une quelconque des revendications précédentes, qui contient en outre au moins un monomère polymérisable par voie radicalaire, de préférence au moins un (méth)acrylate mono- ou multifonctionnel, de façon particulièrement préférée au moins un diméthacrylate ou un mélange de mono- et diméthacrylates.

10. Matériau dentaire selon la revendication 9, qui contient en tant que monomère polymérisable par voie radicalaire du (méth)acrylate de méthyle, éthyle, 2-hydroxyéthyle, butyle, benzyle, tétrahydrofurfuryle ou isobornyle, du phénoxyéthylèneglycolméthacrylate de p-cumyle (CMP-1E), du méthacrylate de 2-(2-biphényloxy)-éthyle, du diméthacrylate de bisphénol-A, du bis-GMA (un produit d'addition d'acide méthacrylique et d'éther diglycidylique de bisphénol-A), du diméthacrylate de bisphénol-A éthoxylé ou propoxylé, du 2-[4-(2-méthacryloyloxyéthoxyéthoxy)phényl]-2-[4-(2-méthacryloyloxyéthoxy)phényl]propane) (SR-348c), du 2,2-bis[4-(2-méthacryloxypropoxy)phényl]propane, de l'UDMA (un produit d'addition de méthacrylate de 2-hydroxyéthyle et de 2,2,4-triméthylhexaméthylène-1,6-diisocyanate), du V-380 (un produit d'addition d'un mélange de 0,7 mole de méthacrylate de 2-hydroxyéthyle et 0,3 mole de méthacrylate de 2-hydroxypropyle avec 1 mole de diisocyanate d'α,α,α',α'-tétraméthyl-m-xylylène), du diméthacrylate de di-, tri- ou tétraéthylèneglycol, du triméthacrylate de triméthylolpropane, du tétraméthacrylate de pentaérythritol, du di- et du triméthacrylate de glycérol, du diméthacrylate de 1,4-butanediol, du diméthacrylate de 1,10-décanediol (D₃MA), du bis(méthacryloyloxyméthyl)tricyclo-[5.2.1.02,6]décane (DCP), un diméthacrylate de polyéthylèneglycol ou polypropylèneglycol, du diméthacrylate de polyéthylèneglycol-200 ou polyéthylèneglycol-400 (PEG-200- ou PEG-400-DMA), du diméthacrylate de 1,12-dodécanediol ou un mélange de ceux-ci.

11. Matériau dentaire selon la revendication 9 ou 10, qui contient en outre au moins un monomère polymérisable par voie radicalaire contenant des groupes acides, de préférence un acide carboxylique, acide phosphorique, apte à la polymérisation, un ester d'acide phosphorique apte à la polymérisation ou un anhydride de ces substances.

12. Matériau dentaire selon l'une quelconque des revendications précédentes, qui contient en outre au moins une charge organique ou inorganique, de préférence un oxyde, tel que SiO₂, ZrO₂ et TiO₂ ou un oxyde mixte à base de SiO₂, ZrO₂, ZnO et/ou TiO₂, une charge microfine ou nanoparticulaire, telle que la silice pyrogénée ou la silice précipitée, de la poudre de verre, telle que de la poudre de quartz, de la poudre de vitrocéramique ou de la poudre de verre opaque aux rayons X, de préférence de la poudre de verre d'aluminosilicate de baryum ou strontium, une charge opaque aux rayons X, telle que le trifluorure d'ytterbium, l'oxyde de tantale-(V), le sulfate de baryum, un oxyde mixte de SiO₂ avec l'oxyde d'ytterbium-(III) ou l'oxyde de tantale-(V), un prépolymère broyé ou un polymère en perles.

13. Matériau dentaire selon l'une quelconque des revendications précédentes, qui contient
(a) 0,01 à 10 % en poids, de préférence 0,05 à 8,0 % en poids et de façon particulièrement préférée 0,1 à 3,0 % en poids d'hydroperoxyde de formule (I),
(b) 0,001 à 5,0 % en poids, de préférence 0,003 à 4,0 % en poids, de façon particulièrement préférée 0,005 à 3,0 % en poids de thiourée et/ou dérivé de thiourée,
(c) 5 à 95 % en poids, de préférence 10 à 95 % en poids et de façon particulièrement préférée 10 à 90 % en poids de monomère polymérisable par voie radicalaire,
(d) 0 à 85 % en poids de charge, et
(e) 0,01 à 5 % en poids, de préférence 0,1 à 3 % en poids et de façon particulièrement préférée 0,1 à 2 % en poids d'additif,
chaque fois par rapport à la masse totale du matériau.

14. Matériau dentaire selon la revendication 13, qui contient 50 à 85 % en poids ou 10 à 70 % en poids de charge.

15. Matériau dentaire selon l'une quelconque des revendications précédentes, destiné à l'utilisation thérapeutique, de préférence en tant que ciment dentaire, composite d'obturation ou matériau de placage.

16. Utilisation non thérapeutique d'un matériau dentaire selon l'une quelconque des revendications 1 à 13, pour la fabrication ou la réparation de restaurations dentaires, telles que prothèses, dents artificielles, inlays, onlays, couronnes, bridges et prothèses complètes.
